# EUROPEAN PATENT APPLICATION

(11) **EP 2 482 216 A2**
(43) Date of publication of application: **01.08.2012**
(21) Application number: 12152184.3
(22) Date of filing: 04.09.2007
(51) Int. Cl.: G06F 19/18

(54) **Methods and systems for designing animal food compositions**

(30) Priority: 01.09.2006 US 469565
(62) Divisional of application: 07841777.1
(71) Applicant: Hill's Pet Nutrition, Inc., Topeka, KS 66603 (US)
(72) Inventor: Al-Murrani, Samer Waleed, Khedheyer, Topeka, KS 66614 (US)
(74) Representative: Daniels, Jeffrey Nicholas

(57) **Abstract**

A method for preparing a food composition for animals comprising (a) accessing at least one database that comprises a first data set relating functional genomic profile of a biofluid or tissue sample from an animal to physiological condition and optionally genotype of the animal; (b) accessing at least one database that comprises a second data set relating to effects of bioactive dietary components on functional genomic profile; (c) by use of an algorithm drawing on these data sets, processing input data defining physiological condition and optionally genotype of a subpopulation of animals to derive a nutritional formula promoting wellness of one or more animals of the subpopulation; and (d) preparing a food composition based on the nutritional formula.

## Description

### CROSS-REFERENCE TO RELATED APPLICATIONS

This application claims priority to United States Patent Application Serial No. 11/469,565 filed September 1, 2006, which is a continuation-in-part of United States Patent Application Serial No. 11/366,655 filed March 2, 2006 which claims the benefit of United States Provisional Application Serial No. 60/657,980, filed March 2, 2005, the disclosures of which are hereby incorporated by reference herein.

### BACKGROUND OF THE INVENTION

### Field of the Invention

The invention relates generally to animal nutrition and particularly to methods and systems for designing food compositions for animals, including food compositions that promote the health and wellness of defined animal subpopulations.

### Description of the Related Art

Bioactive dietary components (BDCs) that, when included in an animal's diet at an appropriate level, promote wellness of the animal are well known and are now commonly included in pet food products and supplements. Examples of such BDCs include amino acids, simple and complex sugars, vitamins, cofactors, antioxidants, omega-3 fatty acids, various botanical preparations, etc. Recent advances in understanding of some of the roles BDCs play in wellness of dogs and cats, together with discovery of new BDCs, have led to a proliferation of pet food products on the market that are designed or claimed to address particular needs and particular states of health or disease states of different classes of animals.

Paradoxically, this proliferation has not made it easier for a dog or cat owner to select the pet food products, if they exist, that will provide optimum nutrition for his or her pet. Many animals have a plurality or complex of wellness issues, and focusing a feeding plan on one issue, for example obesity or diabetes, while failing to address other issues can be detrimental to the overall wellness and quality of life of the animal.

Various attempts have been made to simplify the matter of selection of a pet food. In the case of dog foods, for example, formulas have long been available for puppies, adult dogs and mature dogs; for active and inactive dogs; for dogs of small and large breeds, and so on.

Methods for customizing a food product to a specific animal have also been proposed. For example, U.S. Patent No. 6,493,641 proposes that a pet owner provide an individual pet profile (e.g., in response to a questionnaire) via an electronic interface, and submit a biological sample (e.g., saliva, stool or hair); from the information thus provided, a customized food formula for the pet is said to be generated.

Swanson et al. (2003) J. Nutr. 133, 3033-3040 predicted that functional genomics of dogs and cats would emerge as important areas of study, and that resources such as dog and cat genome maps "can be applied at the field of nutritional genomics and proteomics, enhancing our understanding of metabolism and optimizing companion animal nutritional health status." *Id*., p. 3033, Abstract. The authors further predicted that "[n]utritional genomics, proteomics and metabolomics will be important in the determination of nutrient requirements of dogs and cats at different life stages, the prevention and treatment of various disease states, and the testing of numerous functional ingredients and herbal supplements that are making their way into the pet food market." *Id*., p. 3038.

Nutritional requirements have hitherto been established mainly by empirical studies involving feeding different compositions to groups of animals according to defined protocols. Data generated from such studies have significantly advanced the art, but there remains a need for improved methods of designing pet foods meeting the wellness needs of specific animal subpopulations, whether defined by genotype, phenotype or a combination of both, including subpopulations defined as individual animals.

### SUMMARY OF THE INVENTION

In various aspects, the present invention provides a series of methods and systems wherein an important component is the processing of information relating to the functional genomic profile (FGP) of animals, particularly companion animals such as cats and dogs.

In one aspect, the invention provides a method of selecting a food composition for an animal subpopulation. The method comprises (a) accessing at least one database that comprises a first data set relating FGP of a biofluid or tissue sample from an animal to physiological condition and optionally genotype of the animal; (b) accessing at least one database that comprises a second data set relating to effects of BDCs on FGP; and (c) by use of a first algorithm drawing on the first and second data sets, processing input data defining physiological condition and optionally genotype of the subpopulation to derive a nutritional formula useful for selecting and preparing a food composition for an animal subpopulation. In one embodiment, the method further comprises preparing a food composition based upon the nutritional formula. In another aspect, the invention provides a food composition prepared by the method. The method, nutritional formula, and food composition are useful for promoting wellness and/or for preventing or treating disease in one or more animals of the subpopulation.

In another aspect, the invention provides a computer-aided system for designing a nutritional formula for an animal subpopulation. The system comprises on one to a plurality of user-interfaceable media (a) a first data set relating FGP of a biofluid or tissue sample from an animal to physiological condition and optionally genotype of the animal; (b) a second data set relating to effects of BDCs on FGP; and (c) a first algorithm capable, while drawing on the first and second data sets, of processing input data defining physiological condition and optionally genotype of the subpopulation to derive a nutritional formula promoting wellness of one or more animals of the subpopulation.

In a related aspect, the invention provides a method of designing a nutritional formula for an animal subpopulation. The method comprises accessing the computer-aided system described above to derive, via the first algorithm thereof, a nutritional formula promoting wellness of one or more animals of the subpopulation.

In a further aspect, the invention provides a method of promoting wellness of an animal subject that is a member of a subpopulation. The method comprises (a) accessing at least one database that comprises a first data set relating FGP of a biofluid or tissue sample from an animal to physiological condition and optionally genotype of the animal; (b) accessing at least one database that comprises a second data set relating to effects of BDCs on FGP; (c) by use of a first algorithm drawing on the first and second data sets, processing input data defining physiological condition and optionally genotype of the subpopulation to derive a nutritional formula promoting wellness of one or more animals of the subpopulation; (d) preparing a food composition based on the nutritional formula thus derived; and (e) feeding the food composition to the subject.

In a further aspect, the invention provides a method of prescribing a wellness diet for an animal subject that is a member of a subpopulation definable by genotype and/or physiological condition. The method comprises (a) accessing at least one database that comprises a first data set relating FGP of a biofluid or tissue sample from an animal to physiological condition and optionally genotype of the animal; (b) accessing at least one database that comprises a second data set relating to effects of BDCs on FGP; (c) by use of an algorithm drawing on the first and second data sets, processing input data defining physiological condition and optionally genotype of the subpopulation to derive a nutritional formula promoting wellness of one or more animals of the subpopulation; and (d) prescribing a diet for the subject based on the nutritional formula thus derived.

In one aspect, the invention provides a method of selecting a nutritional formula for use by an animal subject, preferably a companion animal subject. The method comprises (a) accessing at least one database that comprises a test data set (sometimes referred to herein as a "second" data set) relating to effects of BDCs on FGP and (b) by use of an algorithm (sometimes referred to herein as a "first" algorithm) drawing on the test data set, processing input data that define a baseline FGP for the subject to derive a nutritional formula. The formula so derived, in a situation where the baseline FGP is normal, promotes at least maintenance of a normal FGP; and in a situation where the baseline FGP is extranormal, promotes a shift of FGP towards normality. Optionally, this method further comprises accessing at least one database that comprises a sample data set (sometimes referred to herein as a "first" data set) from which normal and extranormal FGPs can be identified for animals having ranges of genotype and physiological condition that encompass the genotype and physiological condition respectively of the subject. In this case the algorithm draws on both the sample data set and the test data set in processing the input data.

In a further aspect, the invention provides a method of diagnosing a state of wellness, disease or physiological disorder, or a predisposition to disease or physiological disorder, in an animal subject, preferably a companion animal subject. The method comprises (a) accessing at least one database that comprises a sample data set from which normal and extranormal functional genomic profiles can be identified for animals having ranges of genotype and physiological condition that encompass the genotype and physiological condition respectively of the subject; and (b) by use of an algorithm drawing on said test data set, processing input data that define a functional genomic profile for the subject to derive a diagnosis. Optionally a treatment or prophylaxis can be prescribed, based upon the diagnosis thus derived.

In a still further aspect, the invention provides a data bank comprising one to a plurality of media residing on or linked electronically to a computer. The media have stored therein or thereon data relating functional genomic profile of an animal species or model to at least one of (a) physiological condition and optionally genotype of an animal providing one or more tissue and/or biofluid samples from which the functional genomic profile is determined and (b) exposure of the animal species or model to one or more bioactive dietary components. The data according to this aspect are configured as one to a plurality of databases from which, on submission of a query relating to functional genomic profile and/or bioactive dietary components via the computer, information in pertinent response to the query is retrievable.
1. Another aspect of the invention provides for a less invasive method for predicting an animal's physiological state, predisposition to disease or its ability to respond to treatment without relying on the use of solid tissue obtained from the animal. The method comprises taking biofluid samples from animals with defined physiological conditions (e.g. control vs. disease), determining the genomic, proteomic and metabolomic profiles that reflect the physiological condition, and employing learning algorithms, such as but not limited to, Weighted Voting, Class Neighbors, K-Nearest Neighbors and Support Vector Machines to define a group of genes, gene products or metabolites from within those profiles that can unambiguously recognize and differentiate between the different physiological conditions under question. This refined profile or "class predictor" can subsequently be used to determine a new or unknown animal's physiological state, predisposition to disease or its ability to respond to treatment without relying on the use of solid tissue obtained from the animal. Thus, for example, it is contemplated herein that an aspect of the invention may include a method for predicting the "physiological" class or condition of an animal and its propensity to develop disease or to respond to a given nutritional treatment comprising:
   a) applying supervised learning algothrims to genomic, proteomic and/or metabolomic data obtained from a learning set of animals or samples that exhibit different physiological states;
   b) determining a class prediction rule;
   c) applying the class prediction rule to a new set of test samples;
   d) classifying or assigning membership of the test samples and therefore the animal that provided the sample, to a particular physiological state based on the class prediction outcome resulting from step (c); and
   e) using the results of step (d) to determine means for bringing an animal from an abnormal physiological state to a normal physiological state using BDCs incorporated into diets. It is further contemplated that this method may comprise an additional step (f) comprising using the class prediction rule to follow the animal's response to the treatment described in step (e) of said method.

Additional and further objects, features, and advantages of the present invention will be readily apparent to those skilled in the art.

### BRIEF DESCRIPTION OF THE DRAWINGS

Figure 1 is a representation to help visualize shifts in FGP from a normal to an extranormal state due, for example, to a disease or physiological disorder, and from an extranormal to a more normal state by practice of a method of the invention.

Figure 2 shows, in much simplified diagrammatic form, some of the processes involved in gene expression, protein function and metabolism in an animal cell (A) before and (B) after intervention of a BDC.

Figure 3 is an illustrative process flow chart of a method of the invention.

### DETAILED DESCRIPTION OF THE INVENTION

The present invention provides methods and compositions for improving the health and/or well-being of an animal, in particular a companion animal such as a dog or a cat.

The nutrition and health of animals are among the most important aspects of pet care. Many animal owners have difficulty in determining if an animal is receiving a well balanced and healthy diet. While people are becoming much more aware regarding their own personal nutrition, there is relatively little knowledge of the advanced dietary requirements essential for the health and well-being of animals.

Canine and feline foods now include formulations based on age, size, body composition, breed and other characteristics of pets, and are designed to address specific differences, for example between different breeds or breed sizes. Formulations can be based on phenotypic differences such as growth rate in large-breed versus smaller dogs. See, for example, U.S. Patent Nos. 5,851,573, 6,156,355, and 6,204,291.

The greater growth rate in large-breed dogs can lead to orthopedic failure (e.g., hip dysplasia) due to impaired bone development caused by rapid muscle growth. Dogs represent a large diversity of phenotypic characteristics. Some formulations have been designed specifically for an individual animal based on the phenotypic characteristics of the specific animal. See, for example, U.S. Patent No. 6,669,975.

Dog breeds have traditionally been grouped on the basis of their roles in human activities, their physical phenotypes, and historical records. Currently more than 400 breeds of dogs are described in the world today, with about 152 of these breeds recognized by the American Kennel Club (AKC) of the United States. Over 350 genetic disorders of purebred dogs have been described, and many of these are restricted to a specific breed, breed type or genetic disposition. See Patterson et al. (1998) J. Am. Vet. Med. Assoc. 193(9), 1131-1144. Many of these mimic common human disorders and their restriction to particular breeds or groups of breeds is believed to be a result of aggressive breeding programs used to generate specific morphologies.

Recently, a new branch of genomic research in humans has emerged that is the interface between nutritional environment and cellular and genetic processes. Such genomic research is referred to as nutritional genomics or "nutrigenomics". Nutrigenomics seeks to provide a genetic understanding for how dietary components and/or nutrition affect the balance between health and disease, for example by altering the expression and/or structure of an individual's genetic makeup. Some dietary components have been shown to alter gene expression in a number of ways. For example, they may act as ligands for proteins such as transcription factors or receptors, may be metabolized by primary or secondary metabolic pathways, thereby altering concentrations of substrates or intermediates, or may be involved in signal pathways.

Functional Genomic Profile: The term "phenotype" as used herein refers to the totality, or any part thereof, of observable characteristics, whether functional or otherwise, of an organism as determined by the genotype of the organism. The term "genotype" refers to the total genetic constitution of an organism, or any part thereof. The genotype comprises genetic information carried both in chromosomes and extrachromosomally.

The term "functional genomic profile" herein refers to the whole or any part of the functional consequences of expression of gene sequences, including production and function of mRNAs, proteins and metabolites. A functional genomic profile (FGP) can be established using genomic, proteomic or metabolomic approaches, or any combination of these.

FGP for the purpose of the present invention can be defined as a pattern of two or more polynucleotides (DNA or RNA), peptides, proteins, metabolites, biomarkers, SNPs (particularly functional SNPs) or combinations thereof, such pattern being associated with a physiological condition of an animal or with response of an animal model to exposure to one or more BDCs.

In some instances an FGP, in whole or in part, contains genes, proteins or metabolites that are the actual cause or that contribute causally to a disease or disorder. In such instances, therapeutic intervention directed against the FGP or the part of the FGP that is a cause of an abnormality can be effective in treating the abnormality.

In other instances, an FGP that is not causally involved in a disease or disorder can still be associated with the disease or disorder such that the FGP can be used as an indicator for the disease or disorder, before and after therapy. By monitoring the FGP in such an instance, success or otherwise of therapy can be established.

Genomic, proteomic and/or metabolomic data that constitute an FGP can be generated from biofluid and/or tissue samples by any technique known in the art of functional genomics. Examples of techniques useful in generating functional genomic analysis include, without limitation, the following techniques that can be used individually or in combination:
(a) single and multicolor gene and protein arrays and microarrays in low and high density formats, for example on glass, silica, plastic, membrane or bead supports or combinations thereof, including for example Northern blot analysis and Western blot analysis; (b) mass spectrometry techniques using quadrupole, time of flight, quadrupole ion trap or Fourier-transform ion cyclotron resonance mass spectrometers or combinations thereof, with various ionization sources including without limitation matrix-assisted laser desorption ionization, electrospray ionization, nanospray ionization and surface-enhanced laser desorption ionization; (c) polymerase chain reaction (PCR) techniques including single and multiplexed quantitative real-time PCR techniques; (d) gel electrophoresis (single or multidimensional) including two-color 2D gel methodologies, SDS-polyacrylamide gel electrophoresis (SDS-PAGE), and 2D PAGE; and (e) liquid chromatography (single or multidimensional) by itself or in tandem with mass spectrometry techniques.

FGPs can be generated from raw image, numerical and/or text data sets, typically after normalization and pre-processing to reduce or remove data noise. Techniques that can be used to recognize FGPs include without limitation nearest neighbor pattern recognition, neural networks, hidden Markov models, Bayesian networks, genetic algorithms, support vector machines, and combinations thereof.

The use of a FGP makes the present systems and methods much more powerful than methods previously proposed that rely only on genomic or genotypic analysis. The number of functional proteins in a cell of an organism far exceeds the number of DNA sequences that represent individual genes in the cell. This is due to the fact that an individual gene sequence can give rise to more than one functional protein. Gene sequences can be modified by addition, deletion or polymorphism of one or more nucleotides on one or both alleles at a gene locus, and this is further complicated by a mechanism of alternative splicing that can give rise to more than one mRNA species from each individual gene sequence, thereby resulting in many different forms of protein.

The apparent discrepancy between number of genes and number of functional proteins is further compounded by the fact that a protein can be modified by one or more post-translational modifications that can include proteolytic cleavage, phosphorylation, glycosylation, acylation, methylation, sulfation, prenylation, vitamin C-dependent modifications (*e.g*., proline and lysine hydroxylation and carboxy terminal amidation), vitamin K-dependent modifications (*e.g*., carboxylation of glutamic acid residues) and incorporation of selenocysteine to form selenoproteins.

The FGP of an animal, or an animal model, or of a tissue or cell thereof, can be in a "normal" or "extranormal" state. A "normal" FGP is one occurring in an animal exhibiting a state of wellness as defined herein, and generally indicative of such a state. Typically a "normal" FGP is associated with homeostasis, *i.e.*, a tendency to stability in bodily functions arising, for example, from internal control systems activated by negative feedback. An "extranormal" FGP is one that is outside the range identified as "normal". An "extranormal" FGP can be associated with a breakdown in homeostasis; thus there is often a tendency for an "extranormal" FGP to drift further from normality with the passage of time, absent intervention (for example by a method of the present invention) to halt or reverse such drift. If unchecked, this progressive drift away from normality can ultimately lead to death. An "extranormal" FGP is therefore often indicative of a state adverse to wellness, for example a state of disease or physiological disorder, in an animal. Such a state can be outwardly evident, or can be latent (*i.e.,* asymptomatic). An "extranormal" FGP can, in some situations, indicate a predisposition, whether hereditary or otherwise, to disease, and in such situations a shift in FGP towards a more normal state (for example by a method of the present invention) can be effective in disease prevention or prophylaxis.

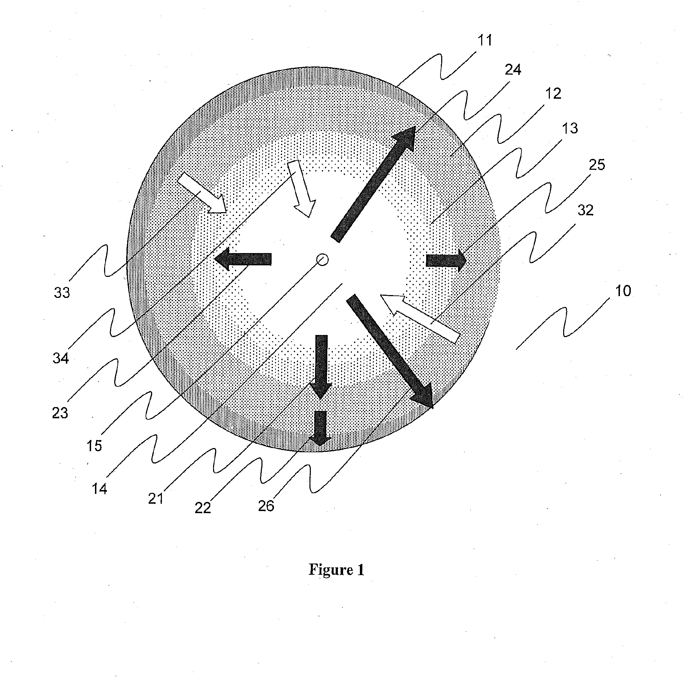

Illustratively and figuratively, Figure 1 shows an FGP domain 10 having an inner circle 14 representing a "normal" FGP. A small region 15 at the center of the inner circle 14 can be considered an "optimum" or "perfect" FGP, but it is emphasized that homeostasis and a state of wellness is generally consistent with any FGP in the "normal" range as represented by the inner circle 14. The domain also has an annular zone 12 representing an "extranormal" FGP, and outside the annular zone 12 an outermost zone 11 where the FGP is so removed from normality that the cell or tissue exhibiting it is in a state of death. There is no sharp dividing line between a "normal" and an "extranormal" FGP, as indicated in Figure 1 by an intermediate or transitional zone 13 between the inner circle 14 ("normal") and the annular zone 12 ("extranormal"). However, if a subject animal is found to have an FGP that is neither clearly "normal" nor clearly "extranormal", *i.e*., in the transitional zone 13, the FGP should be considered "extranormal" and corrective action, for example by a method of the present invention, initiated.

Vectors 21, 22, 23, 24 and 25 represent trends in FGP that occur, for example as an animal enters or progresses in a state of disease or physiological disorder. Vector 22 represents a transition from a disease state to death. Vector 26 represents a transition from a healthy or "normal" state to death. Examples of vector 26 include a healthy animal that is killed in an accident such as being hit by a car. Vectors 32, 33 and 34 represent shifts in "extranormal" FGP resulting from practice of the present invention. Such shifts are, at a minimum, directionally towards a more normal state and, if sustained, can bring the FGP fully back to "normal", *i.e*., into the inner circle 14 in Figure 1. Note that where FGP is already "normal", practice of the invention does not necessarily provide a shift to greater normality, but tends to maintain FGP in the "normal" range.

Class Predictor: The term "class predictor" refers to a genomic, proteomic or metabolomic profile that may be generated using supervised learning methods employing algorithms such as, but not limited to, Weighted Voting, Class Neighbors, K-Nearest Neighbors and Support Vector Machines from a group of pre-defined samples ("the training set") to establish a prediction rule that then can be applied to classify new samples ("the test set").

Animals: The term "animal" means a human or other animal, including avian, bovine, canine, equine, feline, hicrine, murine, ovine, and porcine animals. Preferably, the animal is a companion animal, most preferably a canine or feline such as a dog or a cat. Companion Animals: A "companion animal" herein is a vertebrate animal of any species that is kept by a human owner as a domestic pet, or for work related to sensory abilities or useful behavioral attributes of the animals (for example, hunting dogs, guard dogs, sheepdogs, guide dogs, *etc.).* In most cases the species is mammalian. In the present context an "owner" is a person responsible for looking after, most particularly for feeding, the animal, and does not necessarily hold legal ownership of the animal, and can therefore be, for example, a "keeper" or "guardian" or "caregiver" of the animal. An "owner" herein can be one to a plurality of persons sharing such responsibility, for example members of a family, or a person or persons to whom such responsibility is delegated or entrusted. An important aspect that distinguishes a companion animal from animals in many other situations is that its diet is largely or wholly provided by, and thus can be controlled by, its owner. In this respect a companion animal differs from, for example, grazing or foraging animals. An "end-user" herein, for example of a food composition prepared according to a method of the invention, is typically an owner of a companion animal as defined above.

According to a particular embodiment, the species is one characterized by a high degree of phenotypic and possibly genotypic variation across the species as a whole, but embraces a plurality of breeds, within which there is substantial homogeneity. This is the case, for example, with the domestic dog (*Canis familiaris*) and is applicable to a considerable extent to other species including the domestic cat (*Felis catus*).

The methods of the invention are useful in certain animal species or populations satisfying at least one of the following criteria (a) the animal's diet is largely or wholly controlled by an owner (including a keeper or guardian) and (b) the species or population embraces a plurality of breeds, within which there is substantial phenotypic and possibly genotypic homogeneity; whether or not such species or populations are typically recognized as "companion animals". Thus the present methods are useful in nutritional management of certain farm animals such as chickens and hogs, exotic animals in zoos and parks, and the like.

Subpopulations: A "subpopulation" herein is a set of one to many animals of one species, but less than an entire species, definable in terms of genotype and/or one or more attributes of physiological condition that, in a subpopulation of more than one member, are common to members of the subpopulation.

In certain embodiments, the subpopulation is defined at least in part by breed type. For example, in the case of dogs, various breed types such as gundogs, terriers, toy dogs, hounds, herding dogs, *etc.,* can be identified, each of which comprises a number of specific breeds.

In certain embodiments, the subpopulation is defined at least in part by specific breed. For example, recognized dog breeds (some of which are further subdivided) include afghan hound, airedale, akita, Alaskan malamute, basset hound, beagle, Belgian shepherd, bloodhound, border collie, border terrier, borzoi, boxer, bulldog, bull terrier, cairn terrier, chihuahua, chow, cocker spaniel, collie, corgi, dachshund, dalmatian, doberman, English setter, fox terrier, German shepherd, golden retriever, great dane, greyhound, griffon bruxellois, Irish setter, Irish wolfhound, King Charles spaniel, Labrador retriever, lhasa apso, mastiff, newfoundland, old English sheepdog, papillion, pekingese, pointer, pomeranian, poodle, pug, rottweiler, St. Bernard, saluki, samoyed, schnauzer, Scottish terrier, Shetland sheepdog, shih tzu, Siberian husky, Skye terrier, springer spaniel, West Highland terrier, whippet, Yorkshire terrier, *etc.* In the case of animals of mixed breed, a subpopulation can be defined at least in part by breed heritage, which can be established through knowledge of the parental breeds, phenotypic characteristics, genotypic assessment, or by genetic markers such as SNPs.

In certain embodiments, the subpopulation is defined at least in part by physiological condition. The term "physiological condition" herein refers to any one or combination of physical, pathological, behavioral and biochemical attributes of an animal including its size, weight, age, activity level, disposition, and state of wellness or disease. Physiological condition is a product of interaction of the genotype with the environment of the animal. A subpopulation defined at least in part by physiological condition can cut across breed lines. For example, a subpopulation can consist of adult cats that shed hair excessively, obese dogs, toy dogs having respiratory disease, geriatric dogs of large breed type, long-haired cats having renal insufficiency, *etc*. Alternatively, a subpopulation can be defined in part by physiological condition but restricted to one or a few breeds or a defined breed heritage. Examples of such subpopulations are aggressive poodles, Labrador retrievers with tapeworm infestation, spayed female dogs having a breed heritage that includes beagle, *etc.*

A subpopulation can, in certain embodiments, be very small, for example where members are familially related (*e.g*., offspring of a single stud dog, or kittens of a single litter), or in some embodiments can be defined as an individual animal. In one series of embodiments, the subpopulation is canine. In another, the subpopulation is feline.

Biofluid and Tissue Samples: A biofluid or tissue sample useful herein can be any such sample that is amenable to genomic, proteomic and/or metabolomic analysis. For genomic analysis, the sample must provide DNA in a quantity that may or may not need amplification, for example through PCR techniques. Samples lacking DNA or RNA, as is often the case, for example, with urine samples, can nonetheless provide useful proteomic and/or metabolomic information.

Biofluids that can be sampled include excreta (feces and urine), blood, saliva, amniotic fluid, *etc.* Tissue samples can be obtained *post mortem* from any part of the body of an animal, but for the present purposes more usefully from living animals, for example by biopsy, by surgical removal (*e.g*., during surgery being conducted for other purposes), by cheek swab or by pulling a few hairs.

First Data Set: The systems and methods of the invention, as set forth above, involve at least two data sets, referenced herein as a "first" (or sample) and a "second" (or test) data set. These data sets are typically stored in digital form and are organized in one to a plurality of databases, which are held on user-interfaceable media such as any computer or peripheral memory or data storage device. A database can be "virtual," *i.e.,* existing only through networking of a plurality of devices.

The first and second data sets can be parts of a single database or can be in separate databases. Either or both of the first and second data sets can, if desired, be configured in more than one database, so long as the data can be accessed for processing as discussed more fully below.

The first data set (sample data set) comprises data derived from functional genomic analysis of a multiplicity of biofluid and/or tissue samples obtained from animals representing a wide range of genotypes (*e.g*., canine or feline breeds) and phenotypes or physiological conditions, including healthy animals (typically exhibiting a "normal" FGP) and animals in a variety of disease states (typically exhibiting an "extranormal" FGP). The data are configured relationally, *i.e.*, in such a way as to permit correlation of functional genomic parameters with genotypic and phenotypic attributes. In this way, the first data set can be used to define a functional genomic profile (FGP) for any subpopulation having genotype and physiological condition embraced by the data set. As the first data set increases in extensiveness, a number of beneficial outcomes can be realized: (1) the range of subpopulations embraced by the data set becomes more comprehensive; (2) FGP data for particular subpopulations become more reliable; and (3) the data can be used with greater confidence to develop a predicted FGP for a subpopulation not specifically represented in the data, among other advantages.

Functional genomic analysis of each sample as reflected in the first data set can include analysis with respect to one or more of DNA, RNA (for example mRNA), proteins, metabolites and biomarkers such as enzymes.

To illustrate use of the data to develop a predicted FGP, consider a data set that includes a large volume of functional genomic data on, say, border collies in a variety of states of wellness and disease, but lacking data specifically on border collies having a family history of bladder cancer and showing symptoms of osteoarthritis. If functional genomic data are present that correlate with a predisposition to bladder cancer and with osteoarthritis in other breeds, the data relating to border collies, predisposition to bladder cancer, and osteoarthritis can be processed using well-known statistical techniques to yield a predicted or "best fit" FGP for the subpopulation of interest.

Similarly, an algorithm can be used to predict FGP of a subject animal of mixed breed based on data from purebred animals representing parental breeds or breed types of the mixed breed animal. Thus, in some embodiments, correlating functional genomic data can come from FGP of animals of different breeds or mixes thereof but of the same species.

Data to develop a predicted FGP can come from sources other than from functional genomic analysis of biofluid and/or tissue samples as described above. For example, in some embodiments, the data can come from studies published in the literature. In certain embodiments, the data can be obtained from publicly or commercially accessible data banks, for example accessible through a website. In some embodiments the data can come from mining the genome of the species of the subject animal, and in still other embodiments, homologous functional genomic data can be obtained from species other than the subject animal, such as human, rat or mouse. In certain embodiments, data can be obtained through mining of genomes of species other than that of the subject animal.

For the present method to have usefulness across a wide variety of subpopulations, the first data set must be extensive. Where the method is intended to have application to only a limited range of animal subpopulations, a data set derived from a relatively small number of samples, for example up to about 100, can be useful. However, for most purposes, a much more extensive data set is desirable, derived from samples up to about 1,000 in number, for example up to about 10,000 or more.

Thus, the first data set, in one series of embodiments, is derived from samples collected from a multiplicity of animals representative of a range of genotypes and physiological conditions that broadly embrace the subpopulation of interest without necessarily specifically including that subpopulation.

In certain embodiments, the first data set enables normal and extranormal FGPs to be identified for animals having ranges of genotype and physiological condition that encompass the genotype and physiological condition respectively of an animal subject for which input data are submitted. The word "encompass", with respect to genotype in the present context, means that animals at least of the subject's breed type, and normally of the subject's specific breed, or in the case of a mixed-breed subject, animals having similar breed heritage, are represented in the data set. With respect to physiological conditions in the present context, the word "encompass" means that animals individually and collectively having similar physiological conditions to the subject are represented in the data set, even if the subject's particular combination of physiological conditions are not found in a single animal in the data set. Similarly, the ranges of genotype and physiological condition represented in the first data set are considered herein to "encompass" the genotype and physiological condition of a subject if such genotype and physiological condition are independently found in the data set, even if not in a single animal.

Each sample from an individual animal, to be useful, must be associated with a provenance record that becomes part of the first data set. The provenance record comprises zoographical data relevant to defining the genotype and physiological condition, at the time the sample is collected, of the animal that provided the sample.

The term "zoographical data" herein refers to any and all information, whether quantitative or qualitative, that is gathered on an animal providing a biofluid or tissue sample, from sources other than analysis or experimentation on the sample itself. Sources of zoographical data can include the knowledge base of the owner, captured for example as responses to a questionnaire, veterinary records including those indicative of past and present states of wellness or disease, the animal's pedigree if it has one, biometrics (height, weight, *etc*.) at time of sample acquisition, *etc.*

Zoographical data included in the first data set can comprise one or more data items relating to genotype. Examples of such data items include without limitation: the breed of the animal, whether pedigreed, registered by a body such as the AKC or otherwise; the pedigree if known; in the case of animals of mixed breed, the breed heritage of the animal including the breed(s) of its parents and, if available, ancestors of earlier generations; sex; and coat type (*e.g*., long, short, wiry, curly, smooth) and coloration; evident hereditary conditions and disorders.

Zoographical data included in the first data set can comprise one or more data items relating to physiological condition. Examples of such data items include without limitation: age (chronological and, if determinable, physiological); weight; dimensions (*e.g*., height at shoulder, length of legs, length of back, *etc.*); veterinary medical history; reproductive history, including whether neutered, number and size of litters; present wellness or disease state and any recent changes therein, including any condition or disorder diagnosed, and any symptoms whether or not diagnosis has been made; presence of parasites, including fleas; appetite and any recent changes therein; physical activity level; mental acuity; behavioral abnormalities; and disposition (*e.g*., timid, aggressive, obedient, nervous).

The "chronological age" of an animal is the actual time elapsed (*e.g*., in years or months) since birth. The "physiological age" of an animal is an estimate of the average chronological age of animals of similar breed exhibiting the same age-related physiological condition (mobility, mental acuity, dental wear, *etc.)* as the animal.

Zoographical data can further relate to aspects of the environment in which an animal subpopulation lives. Such aspects include without limitation climate, season, geographical location and habitation. For example, it can be material to developing a food composition for an animal to know whether the animal lives in a warm or dry climate, or an arid or humid climate; whether it is currently spring, summer, autumn or winter; whether the animal is housed indoors or outdoors; whether the animal is in a home, a boarding kennel, a place of work (*e.g*., in the case of guard dogs, police dogs, *etc.)* or some other habitat; whether it is housed alone or with other animals; whether it lives in an urban or rural area; zip code, state and/or country of occupancy; whether and to what extent its habitat is affected by pollutants (*e.g.*, tobacco smoke); and so on.

Second Data Set: The second data set (test data set) comprises data on effects of bioactive dietary components (BDCs), alone and in combinations, on FGP. These data can include publicly or commercially available information from any source and/or results of studies conducted for the express purpose of building the second data set.

Historically, gross effects of particular BDCs on wellness have been determined by feeding studies using live animals of the species of interest, for example dogs or cats. According to the present invention, effects of BDCs at the subcellular level, *i.e.,* on the FGP of cells, can be determined by controlled experiments wherein an animal model is exposed to different levels of, and/or different durations of exposure to, one or more BDCs. "Different levels" of a BDC in the present context include a zero level of the BDC. If multiple levels of a BDC are included in a test, it may be possible to elucidate a dose response.

In certain embodiments, the animal model can be live animals of the species of interest. However, an extensive data set can be more rapidly and economically assembled by use of one or more alternative testing models as exemplified below.

In one embodiment, the alternative testing model is a vertebrate model, for example a small species well adapted to functional genomic studies such as mice, rats, guinea pigs, rabbits or chickens. In another, the alternative testing model is an invertebrate model, for example an invertebrate species such as the roundworm *Caenorhabditis elegans (C. elegans)* or the fruit fly *Drosophila melanogaster,* the genome of which has been substantially elucidated. In a further, the alternative testing model is a non-animal model, for example a yeast such as *Candida albicans.* In another, the alternative testing model is a cell culture model, for example using primary and/or immortalized cell lines from the species of interest (*e.g*., canine or feline) or from another species, including human. In another, the alternative testing model is an *ex vivo* model using tissue explants obtained from an animal and maintained outside the body of the animal.

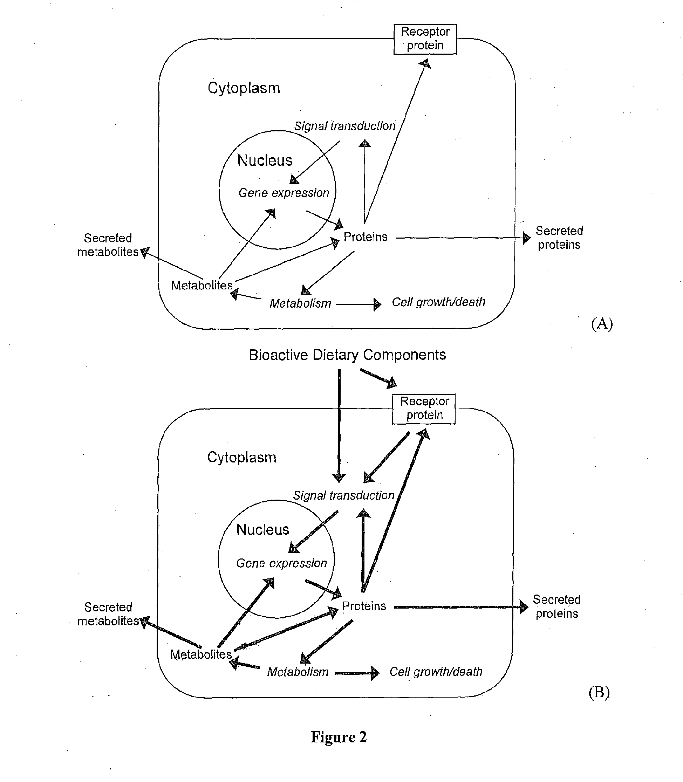

In Figure 2, diagrams (A) and (B) show some of the processes involved in gene expression, protein function and metabolism in an animal cell, respectively before and after intervention of a BDC.

Without being bound by theory, at the cellular level, a BDC may act as a ligand for a protein such as a transcription factor or a receptor, may be metabolized by primary or secondary metabolic pathways, thereby altering concentrations of substrates and/or intermediates involved in gene regulation or cell signaling, or may alter signal transduction pathways and signaling by positively or negatively affecting signal pathways.

The processes of signal transduction, gene expression and metabolism are all mediated by proteins. As shown in Figure 2(B), a BDC can enter the cytoplasm of the cell and, via the signal transduction process, affect gene expression in the nucleus. Alternatively, a BDC can engage a receptor protein at the cell membrane (the outer boundary of the cytoplasm), and the receptor protein sends a signal via the signal transduction process that affects gene expression in the nucleus. Through modification of gene expression, a BDC can affect a great variety of protein-mediated processes, as symbolized in Figure 2(B) by use of bolder arrows than in Figure 2(A).

The second data set can include data not only on chemical or biological entities known as BDCs but on a variety of materials not previously known to have nutritional, nutraceutical or pharmacological effect. All such materials are considered BDCs herein if a useful effect on expression of at least one gene, function of at least one protein or production of at least one metabolite is found. In one embodiment, BDCs of interest herein are materials having GRAS (generally regarded as safe) or equivalent status under U.S. FDA (Food and Drug Administration) regulations or counterpart regulations in other countries, or are eligible for such status. In other embodiments a BDC can be a therapeutically or pharmacologically effective compound, *e.g.*, a drug or herbal medicine.

Many BDCs are chemical entities, generally naturally occurring in foods from which they can be extracted. BDCs can, in many cases, also be prepared by microbiological (e.g., fermentation) or synthetic processes. Examples of BDCs that are chemical entities include without limitation: amino acids; simple sugars; complex sugars; medium-chain triglycerides (MCTs); triacylglycerides (TAGs); n-3 (omega-3) fatty acids including α-linolenic acid (ALA), eicosapentaenoic acid (EPA) and docosahexaenoic acid (DHA); n-6 (omega-6) fatty acids including linoleic acid (LA), γ-linolenic acid (GLA) and arachidonic acid (ARA); choline sources such as lecithin; fat-soluble vitamins including vitamin A and precursors thereof such as carotenoids (*e.g*., β-carotene), vitamin D sources such as vitamin D₂ (ergocalciferol) and vitamin D₃ (cholecalciferol), vitamin E sources such as tocopherols (e.g., α-tocopherol) and tocotrienols, and vitamin K sources such as vitamin K₁ (phylloquinone) and vitamin K₂ (menadione); water-soluble vitamins including B vitamins such as riboflavin, niacin (including nicotinamide and nicotinic acid), pyridoxine, pantothenic acid, folic acid, biotin and cobalamin; and vitamin C (ascorbic acid); antioxidants, including some of the vitamins listed above, especially vitamins E and C; also bioflavonoids such as catechin, quercetin and theaflavin; quinones such as ubiquinone; carotenoids such as lycopene and lycoxanthin; resveratrol; and α-lipoic acid; L-carnitine; D-limonene; glucosamine; S-adenosylmethionine; and chitosan.

With respect to the inclusion of amino acids in the above illustrative list of BDCs, almost all foods contain protein, which typically supplies all essential amino acids. However, the protein content of a food does not necessarily supply essential amino acids in proportions that are optimal for wellness of particular animal subpopulations, thus supplementation with one or more amino acids, or with protein sources rich in such amino acids, can be desirable.

Similar considerations apply in the case of simple and complex sugars that are BDCs and may or may not be components of the carbohydrate fraction of a food, and certain fatty acids, including n-3 and n-6 fatty acids, which are BDCs and may or may not be components of the lipid fraction of a food.

Otherwise the macronutrients required in a balanced animal diet (protein, carbohydrate, fat and fiber) are considered separately from BDCs such as those listed above in designing a nutritional formula, as will be discussed below.

Certain biological materials, especially botanical materials, can be considered BDCs and can, if desired, be included in the second data set. In many of these, a bioactive chemical entity has been identified; even where a bioactive component is known other, unknown, bioactive components may be present and contribute to the bioactive effect of the biological material.

Illustrative botanicals that can be useful as BDCs include, without limitation, aloe vera, dong quai, echinacea, evening primrose, flaxseed, garlic, ginger, ginkgo biloba, ginseng, green tea, soy, turmeric, wheat grass and yerba mate.

The second data set thus comprises data relating FGP effects in an animal model to BDCs tested in the model. From this data set, by use of a suitable algorithm, a BDC or combination of BDCs can be selected having a desired effect on FGP.

Input Data: The input data processed according to methods of the invention comprise data that define the genotype and physiological condition of the subpopulation for which a diet is to be designed, a nutritional formula prescribed or a food composition prepared. The input data can comprise zoographical data, including any of the types of zoographical data mentioned above as part of the provenance record of samples in the first data set. In certain embodiments, input data for an animal subject include FGP data derived from one or more tissue and/or biofluid samples provided by the subject. According to these embodiments, the input data can indicate an FGP in a normal or extranormal range.

A computer-aided system of the invention typically comprises a user interface enabling entry of the input data.

Entry of zoographical input data to the system can be made by an interface operator based on a hard-copy or electronic questionnaire filled out by an owner of an animal subject. Alternatively, entry of such data can be effected at an interface directly by the owner.

The user interface for entry of zoographical data can be remote from a main processor (where the input data are processed according to a method of the invention) but linked thereto via a network such as the internet. Alternatively the user interface can be local (e.g., hard-wired) to a main processor, for example at a retail store or veterinarian's office. In various embodiments the user interface can, illustratively and without limitation, comprise a keyboard and monitor; a personal computer, for example in the owner's home; a touch-screen terminal; a touch-tone telephone; or a voice-activated system. Alternatively, the zoographical data can be pre-entered into a computer-readable medium such as printed barcodes or computer-readable alphanumeric characters; floppy disk; CD-ROM; memory card; chip; etc., and scanned or uploaded at a terminal equipped to read from such a medium. The medium can, in some embodiments, be attachable to the subject animal, for example on a collar, ear-tag or collar-attached dog-tag, or, in the case of certain types of chip, surgically implanted under the animal's skin. As yet another option, the zoographical data can be pre-entered into the computer-aided system itself and stored in a database, whence it can be retrieved by entry of a code unique to the subject animal for which the zoographical data are originally entered. Such a code can be entered via any interface type and on any suitable medium, including those indicated above.

Processing Input Data to Derive a Nutritional Formula: Processing of the input data for a subpopulation (which can, as stated above, be a single animal subject) is accomplished by means of an algorithm, herein sometimes referred to as a "first" algorithm, that draws on the first and/or second data sets described above to derive from the input data a nutritional formula that promotes wellness of one or more animals of the subpopulation.

The algorithm, at least for embodiments of the invention wherein the algorithm draws on both a first and a second data set as described above, can illustratively be embodied in a computer program that incorporates at least the following tasks. Processing does not necessarily occur in the sequence presented below. A computer-aided system of the invention can optionally employ parallel processing, wherein two or more tasks are handled simultaneously.

In one task, input data from a subject are read into memory. In another, a search is conducted of the first data set for zoographical and/or FGP data that correspond as closely as possible to the input data. Searching and statistical techniques known in the art can be used to establish one to a plurality of "hits" that collectively provide a best fit to the genotype and physiological condition of the subject. Where an FGP is not available for the subject, the algorithm computes an FGP corresponding to zoographical input data, and identifies any departure from a normal state that may exist in the FGP. Where an FGP is provided as part of the input data for the subject, the algorithm again identifies any departure from a normal state. In a further task, a search is conducted of the second data set for test data pertaining to an FGP as established above for the subject. Test data indicating a BDC or combination of BDCs that are effective to maintain such an FGP in a normal state, or shift such an FGP from an extranormal state towards greater normality, are retrieved, for example using searching and statistical techniques known in the art to provide a best fit to the FGP of the subject. In another task, a nutritional formula is computed incorporating effective amounts of one or more BDCs identified as described above. The nutritional formula can be computed in the form of a complete diet, incorporating basic energy, protein and fiber requirements (which can be readily established from the input zoographical data) together with the identified BDCs. Alternatively, the nutritional formula can be computed in the form of a dietary supplement, excluding basic energy, protein and fiber requirements.

Optionally, the nutritional formula can be output via a user interface such as a computer video screen, printer, voice synthesizer, etc. A code representing the nutritional formula can be downloaded to a user-readable or computer-readable medium, for example a printed barcode, a printed numerical code, the data strip of a card, a memory device, a disk, a chip etc. In one embodiment such a code is downloaded to a chip adapted for implantation in an animal, particularly a companion animal.

In embodiments where the nutritional formula is further processed, for example to generate a food composition, no output of the nutritional formula itself may be required.

In certain embodiments, a food composition is formulated directly by amalgamating the first algorithm with a second (formulation) algorithm as described below. According to such embodiments, computation of a nutritional formula as an intermediate step may or may not occur.

Where a nutritional formula is output, BDCs and other components can be expressed in any suitable form. For example, components can be expressed in terms of their content in a food composition (e.g., in % or in mg/g, usually on a dry matter basis), in terms of a daily dosage or allowance (e.g., in g/day), optionally on a live weight basis (e.g., in mg/kg/day). An illustrative specimen nutritional formula that may be generated by practice of the present invention is shown in Table 1.

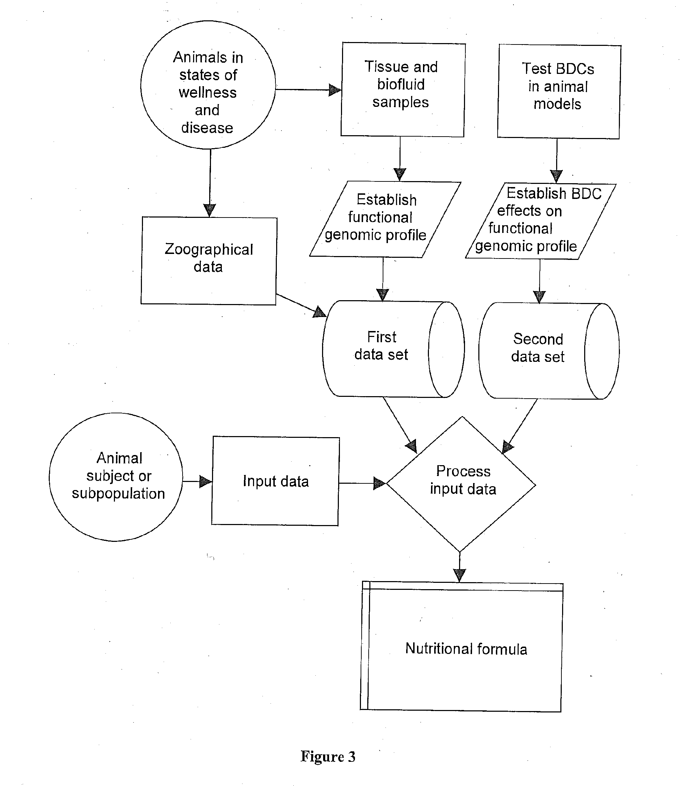

Overview of an Illustrative Method of the Invention: Figure 3 is a flow chart showing an illustrative method for designing a nutritional formula. According to this illustrative method, a key step is the processing of input data, shown as a diamond in Figure 3, to generate the nutritional formula, for example as described immediately above. Three subsystems feed into the processing step.

In a first subsystem, starting at the top left of Figure 3, animals in various states of wellness and disease are identified. As suggested above, it is often desirable that this set of animals be as large as possible, and include as broad as possible a range of states of disease and physiological disorder as well as a broad range of genotypes. A set of zoographical data is collected for each animal. Each animal is a source of one to a plurality of tissue and/or biofluid samples. Each sample is subjected to functional genomic analysis (including one or more of gene expression, proteomic and metabolomic analysis), for example using an established microarray technique, to establish an FGP for the animal that provided the sample, reflecting the genotype and physiological condition of the animal at the time the sample was collected. Data defining the FGP become part of the first data set as defined herein, in association with the zoographical data relating to the animal.

In a second subsystem, starting at the top right of Figure 3, BDCs are tested in one or more animal models as described above. The more BDCs that are tested, the better; and the more dosages of each BDC tested, the better. Testing can include combinations of BDCs as well as individual BDCs. From all this testing, BDC effects on FGP of the animal model can be established. The test results go to make up the second data set as defined herein.

The first and second data sets are typically organized in one or more relational databases that are adapted for search and retrieval of information by the first algorithm as it processes input data.

In a third subsystem, in the lower left portion of Figure 3, input data are entered for an animal subject or subpopulation. As indicated above, the input data typically include zoographical data and may or may not include FGP data. Processing the input data to generate a nutritional formula requires the processing algorithm to access the first and second data sets as shown in Figure 3. The nutritional formula generated is one that the data stored in the system show or suggest will promote wellness of the subject animal or one or more animals of the subpopulation. Some aspects of "promoting wellness" are described in more detail herein. Optionally (not shown in Figure 3), input data that comprise both zoographical and FGP data are added to the first data set, and can be accessible in future iterations of the method.

In one embodiment, such input data are associated with an identifier or code for the specific animal to which they relate. If the same animal is the subject of a subsequent iteration of the method, the data processing algorithm can be programmed to retrieve prior FGP data for that animal. In this way, trends and changes in FGP of the animal can be tracked. Among other benefits, such tracking can enable periodic monitoring of the effectiveness of the nutritional formula in maintaining a normal FGP, in shifting an extranormal FGP towards greater normality, and/or in any aspect of the promotion of wellness as more fully described herein.

Iterative use of the method for a specific animal can form a basis for a nutritional plan that is monitored throughout all or a substantial part of the life of the animal. Corrective action can be taken by adjusting the nutritional formula whenever the animal's state of wellness declines or its FGP moves into an extranormal state.

Preparing a Food Composition: The end-product of one embodiment of the invention is the nutritional formula derived as set forth above. For example, a veterinary physician or dietician can prescribe a nutritional formula for a subject animal by a method as herein described. A nutritional formula can be designed to provide a total solution to a state of disease or physiological disorder, or it can be adapted for use in conjunction with pharmaceutical (e.g., administration of a drug or other medication) or surgical intervention.

In another embodiment, the nutritional formula is used as the basis for preparing a food composition, which becomes the end-product of this embodiment. A second or formulating algorithm can be used to derive a food composition from the nutritional formula. As mentioned above, such an algorithm can be integrated with the first algorithm to generate a food composition directly by processing the input data. Disclosure herein of a nutritional formula as an intermediate stage in generating a food composition does not limit the present invention to methods and systems wherein such a stage is identifiable.

Algorithms for formulating food compositions based on a nutritional formula as illustrated for example in Table 1 are well known in the art. Such algorithms access a data set having analysis of various food ingredients and draw on that data set to compute the amounts of such ingredients in a food composition having the desired nutritional formula. An illustrative specimen food composition generated by a method of the invention is presented in Table 2.

Optionally the data set on which the second algorithm draws further includes cost data for the various food ingredients, and the second algorithm incorporates a routine to include cost as a criterion in selection of ingredients. This can enable a food composition to be prepared at reduced cost, for example at lowest cost consistent with providing the desired nutritional formula.

Other criteria can be built in if desired. For example, ingredients can be identified as "organic" or otherwise, so that if an "organic" food product is desired only "organic" ingredients are selected.

In one embodiment, the food composition can be selected, from a range of pre-existing options, e.g., an existing pet food product line, to best fit or match the nutritional formula derived by practice of the invention. For example, an algorithm can be used that compares a computed food composition or nutritional formula with those of available products, and selects the product coming closest to matching that composition or formula.

In another embodiment, a pet food is manufactured according to the composition derived as set forth above. Such a pet food is accordingly customized to an individual animal providing the input data, or to an animal subpopulation represented by an animal providing the input data. Such manufacture can be offline, *i.e*., not controlled by a computer-aided system. Alternatively, such manufacture can be in part or in whole under the control of, and/or driven by, an extension of the computer-aided system that generates the nutritional formula and computes a composition for the food as described above.

The product thus manufactured can be a complete food or a supplement adapted for addition to or mixing with a base food to form a complete food. The product can be liquid, semi-solid or solid; if solid, it can be moist (*e.g*., a retortable moist pet food), semi-moist or dry (e.g., a kibble). A supplement can be designed for use, for example, as a gravy to accompany a base food, or as a coating for a base kibble.

Suitable computer-controlled apparatus for manufacturing a food product having a defined composition is known in the art. Illustratively, apparatus substantially as described in U.S. Patent No. 6,493,641 can be used.

Optionally, the food, once prepared according to a method of the invention, is packaged in a suitable container. For example, a moist food can be packaged in a can, ajar or a sealed pouch; a dry food can be packaged in a bag, a box, or a bag in a box. This step can, if desired, also be under control of a computer-aided system.

A computer-aided system of the invention can be further harnessed to print a label or package insert for the food product, having any or all information required by governmental regulations and by customary commercial practice. For example, the label or package insert can include a list of ingredients and/or a guaranteed analysis.

Food manufacture, including packaging and labeling, can occur at a conventional manufacturing site such as a factory. Alternatively, it can be convenient to arrange for manufacture of the food to take place more locally to the end-user, for example at a point of sale at a distributor's or retailer's premises, such as a pet food store. In one embodiment the food composition is prepared at a distribution site and delivered to the end-user, for example in response to an order placed by the end-user, such as by telephone or via a website accessed through the internet.

The food composition is, in one embodiment, prepared by a compounder on receipt of a prescription from a veterinary physician or dietician setting forth the nutritional formula derived by the first algorithm.

In another embodiment, an end-user at a point of sale terminal enters a code representing a nutritional formula previously selected for a specific animal, for example by swiping a card or scanning a chip containing such a code. A computer-aided mixing apparatus, for example a mixing and vending apparatus located at the point of sale, then prepares a food composition based on the nutritional formula thus encoded, and delivers it to the end-user.

A food composition prepared by a method of any embodiment of the present invention is itself a further embodiment of the invention.

Promoting Wellness: The nutritional formula derived from the system or methods of the present invention is one designed to promote wellness of one or more animals of the subpopulation of interest.

"Wellness" of an animal herein encompasses all aspects of the physical, mental and social well-being of the animal, and is not restricted to the absence of infirmity.

"Promoting wellness" herein encompasses maintaining a present state of wellness; preventing occurrence of disease or physiological disorder whether or not the subject animal or subpopulation is predisposed, genetically or otherwise, to such disease or disorder; or, where a state of disease or physiological disorder exists, enhancing any aspect of health. Use of the dual terms "disease" and "physiological disorder" herein does not imply a clear distinction between these terms. Many conditions adverse to wellness, conventionally thought of as diseases, for example diabetes or osteoarthritis, can equally well be considered physiological disorders; likewise conditions conventionally thought of as physiological disorders, for example obesity or halitosis, can equally well be considered diseases. Enhancing health can comprise attenuation and/or elimination of a disease state, including without limitation relief of symptoms, lowering a pathogen or parasite burden, controlling severity of disease within more tolerable limits, and cure, with or without remission.

"Promoting wellness" further encompasses (1) restoring any aspect of FGP, including expression of a gene, function of a protein or production of a metabolite to a more normal state; (2) improving nutritional management of an animal at specific stressful stages in its life, even where no disease or disorder is present, for example during growth and development of a kitten or puppy; during gestation and lactation; before and after surgery, for example spaying; and before, during and after long-distance transportation; and (3) enhancing any aspect of health in offspring of the subject animal or subpopulation, for example by *in utero* nutrition when feeding a gestating female animal.

Conditions adverse to wellness encompass not only existing diseases and physiological (including mental, behavioral and dispositional) disorders, but predisposition or vulnerability to such diseases or disorders. Asymptomatic as well as outwardly evident diseases and disorders are likewise encompassed. The expression "promoting wellness" of an animal is to be understood herein as further encompassing reducing nuisance to humans living in proximity to the animal. Examples of such nuisance include without limitation excessive shedding, odor of excreta including feces, intestinal gas and urine, and allergenicity.

In one embodiment, promoting wellness involves simultaneous prevention, attenuation or elimination of a cluster of two or more disease states in an animal.

Diseases and physiological disorders, whether outwardly evident or latent, for which methods of the invention are applicable, include all such diseases and disorders of the animal species of interest. According to an embodiment of the invention, wellness is promoted by prevention, attenuation or elimination of one or more disease states that are amenable to nutritional management.

Illustratively, in dogs, such diseases and disorders include, without limitation, adverse reactions to food (including food allergy and food intolerance), as can be manifested for example by chronic colitis, chronic gastroenteritis, chronic otitis externa or pruritic dermatitis; arthritis, including osteoarthritis; brain aging and related behavioral changes; cancer or neoplasia; cardiovascular disease, including ascites or edema (fluid retention), heart disease, heart failure, heartworm disease, and primary hypertension; developmental orthopedic disease; diabetes mellitus; gastrointestinal disorders, including colitis, fiber-responsive colitis, fiber-responsive constipation, constipation unresponsive to increased fiber, acute or chronic diarrhea, fiber responsive diarrhea, exocrine pancreatic insufficiency, flatulence, acute or chronic gastroenteritis, inflammatory bowel disease (IBD), maldigestion or malabsorption, non-hyperlipidemic pancreatitis, hyperlipidemic pancreatitis, recovery from gastrointestinal surgery, and acute or chronic vomiting; hepatic disorders, including ascites or edema (fluid retention), copper storage disease, hepatic encephalopathy, and liver disease; lyperlipidemia; obesity; oral health disorders, including gingivitis, oral malodor, and tartar, plaque or dental stain; recovery states, including anemia, anorexia, cachexia or weight loss, convalescence, debilitation, hypermetabolic states, malnutrition, and pre- and post-surgical states; renal disease, including hypertension, renal failure, and renal insufficiency; and urolithiasis, including calcium oxalate, urate and cystine management, struvite dissolution, struvite management, and struvite management in obese prone dogs.

Illustratively, in cats, such diseases and disorders include, without limitation, adverse reactions to food (including food allergy and food intolerance), as can be manifested for example by chronic colitis, eosinophilic granuloma complex, chronic gastroenteritis, or pruritic dermatitis; cardiovascular disease, including ascites or edema (fluid retention), heart disease, heart failure, and primary hypertension; diabetes mellitus; feline lower urinary tract disease, including idiopathic cystitis, oxalate management, struvite dissolution, struvite management, struvite management in obese cats, and struvite management in obese prone cats; gastrointestinal disorders, including colitis, fiber-responsive colitis, fiber-responsive constipation, constipation unresponsive to increased fiber, acute or chronic diarrhea, fiber responsive diarrhea, acute or chronic gastroenteritis, IBD, pancreatitis, recovery from gastrointestinal surgery, and acute or chronic vomiting; hepatic disorders, including ascites or edema (fluid retention), copper storage disease, hepatic encephalopathy, and liver disease; lyperlipidemia; obesity; oral health disorders, including gingivitis, oral malodor, and tartar, plaque or dental stain; recovery states, including anemia, anorexia, cachexia or weight loss, convalescence, debilitation, hypermetabolic states, malnutrition, and pre- and post-surgical states; renal disease, including hypertension, renal failure, and renal insufficiency; and urolithiasis, including calcium oxalate, urate and cystine management, struvite dissolution, struvite management, and struvite management in obese prone cats.

In one embodiment, a method of the invention is repeated at intervals for one or more individual animals of a subpopulation, the nutritional formula being adjusted as needed for changes in physiological condition or FGP over time. Such changes can be brought about at least in part by the nutritional formula(s) of food composition(s) prepared by previous iteration(s) of the method. An iterative method can provide a feeding plan, for example to transition from remediation of a wellness problem to prevention of recurrence of the problem.

Data Bank: The term "data bank" herein refers to a physical embodiment of a collection of data comprising one to a plurality of data sets that can be configured in one to a plurality of databases. A data bank thus comprises a medium wherein or whereon such data are stored. A data bank can comprise more than one such medium; however, in such a case the media are functionally linked. Media useful herein can be user-readable, as in the case of a printed spreadsheet, but typically, and especially in view of the large volume of data presently contemplated, such media are computer-readable.

A data bank of the invention can comprise one to a plurality of media residing on or linked electronically to a computer, the media having stored therein or thereon data relating functional genomic profile of an animal species or model to at least one of (a) genotype and/or physiological condition of an animal providing one or more tissue and/or biofluid samples from which said functional genomic profile is determined; and (b) exposure of the animal species or model to one or more bioactive dietary components. The data are configured as one to a plurality of databases. On submission of a query relating to functional genomic profile and/or bioactive dietary components via the computer, information in pertinent response to the query is retrievable from the one or more databases.

According to one embodiment, such a query requests output of functional genomic profile data relevant to input data on genotype and/or physiological condition of an animal subject. According to another embodiment, such a query requests output of bioactive dietary component data relevant to input data on functional genomic profile of an animal subject. The information retrievable in pertinent response to such a query can be expressible as a nutritional formula for the animal subject.

In a typical data bank of the invention, the data comprise a first data set relating functional genomic profile to genotype and/or physiological condition of an animal providing one or more tissue and/or biofluid samples from which said functional genomic profile is determined; and a second data set relating functional genomic profile to exposure of an animal model to one or more bioactive dietary components. According to this embodiment, information is retrievable in pertinent response to a query requesting output of a nutritional formula for an animal subject appropriate to input data on genotype and/or physiological condition of the subject.

The data in such a data bank optionally further comprise a third data set comprising content of bioactive dietary components in ingredients for a food composition. According to this embodiment, information is retrievable in pertinent response to a query requesting output of a food composition for an animal subject appropriate to input data on genotype and/or physiological condition of the subject.

According to a related embodiment, the third data set further comprises cost of ingredients, and information is retrievable is retrievable in pertinent response to a query requesting output of a cost-optimized food composition for an animal subject appropriate to input data on genotype and/or physiological condition of the subject.

In a data bank of a further embodiment, information is retrievable in pertinent response to a query requesting output relating input data on functional genomic profile of an animal subject to a normal functional genomic profile.

In a data bank of a still further embodiment, information is retrievable in pertinent response to a query requesting output of a nutritional formula for an animal subject effective (a) to maintain a normal functional genomic profile or (b) to modify an extranormal functional genomic profile to a more normal state.

Further embodiments of the invention are those listed below. One such embodiment is a method of selecting a food composition for an animal subject, preferably a companion animal subject. The method comprises (a) accessing a database populated with normal and extranormal functional genomic data; (b) by reference to said data, evaluating the FGP of the subject relative to a normal profile; (c) from a database populated with test results on FGP in an animal model exposed to at least one BDC, identifying one or more BDCs tending to shift FGP to a more normal state; and (d) selecting a food composition comprising said one or more BDCs.

In one aspect of the invention, the food composition is selected with reference to a database populated with data on costs of food ingredients and BDCs. In another, the food composition is effective to shift FGP to a more normal state and is formulated at or below a target cost. In another, the databases are stored on one or more media. In another, a computer capable of accessing the one or more media is used to evaluate functional genomic data. In a further aspect, the method further comprises feeding the composition to the animal subject to prevent development of a disease state in the subject, to enhance the subject's health, to shift the subject's FGP from an extranormal to a normal state, or to effect a change in the subject's FGP. In another, the normal and extranormal functional genomic data are derived from analysis of tissues and/or biofluids of a population of animals in states of wellness and disease. Such a population can be defined at least in part by genotypic parameters, at least in part by phenotypic parameters, or at least in part by breed or group of breeds. In this last instance, the food composition can be selected specifically for the breed or group of breeds.

Another embodiment is a method of formulating a food composition for an animal subject. The method comprises (a) accessing a first data set containing data that relate the subject's FGP as determined from one or more tissue and/or biofluid samples to a normal FGP; (b) accessing a second data set containing information on effects of individual BDCs and/or combinations thereof on FGP in one or more model test systems; and (c) computing a formulation comprising a BDC or combination thereof effective when used as a food composition to reverse or attenuate displacement of the subject's FGP from the normal FGP.

In one embodiment, the formulation is effective to promote a transition of the subject's FGP to a normal FGP. In another, the method further comprises accessing a data set containing information relating to the subject's phenotype. Such information can, for example, be selected from the group consisting of age, coat type, size and weight. In a further aspect, the method further comprises accessing a data set containing information on source and cost of an active form, precursor or metabolite of each BDC in the formulation. In another, the formulation computed by such a method is cost efficient. In another, the normal FGP is established from analysis of tissues and/or biofluids from a population of animals. Such a population can again be defined at least in part by genotypic parameters, at least in part by phenotypic parameters, or at least in part by breed or group of breeds. In this last instance, the food composition can be selected specifically for the breed or group of breeds. In a further aspect, at least one of the data sets is stored on one or more media. In another, a computer capable of accessing the one or more media is used to compute the formulation.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. Similarly, the words "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any compositions, methods, articles of manufacture, or other means or materials similar or equivalent to those described herein can be used in the practice of the present invention, the preferred compositions, methods, articles of manufacture, or other means or materials are described herein.

All patents, patent applications, publications, and other references cited or referred to herein are incorporated herein by reference to the extent allowed by law. The discussion of those references is intended merely to summarize the assertions made therein. No admission is made that any such patents, patent applications, publications or references, or any portion thereof, is relevant prior art for the present invention and the right to challenge the accuracy and pertinence of such patents, patent applications, publications, and other references is specifically reserved.

In the specification, there have been disclosed typical preferred embodiments of the invention and, although specific terms are employed, they are used in a generic and descriptive sense only and not for purposes of limitation, the scope of the invention being set forth in the claims. Many modifications and variations of the invention are possible in light of the above teachings. It is therefore to be understood that within the scope of the appended claims the invention may be practiced otherwise than as specifically described.

### EXAMPLES

The invention can be further illustrated by the following examples, although it will be understood that these examples are included merely for purposes of illustration and are not intended to limit the scope of the invention unless otherwise specifically indicated.

### Materials and Methods

### Isolation of Ribonucleic Acid (RNA) from Tissue

Tissue samples that have been collected, frozen in liquid nitrogen, and thawed are homogenized and processed using a TRIzol® RNA extraction method to produce good quality RNA which is then subjected to further genomic analysis.

Materials: Ice, Liquid nitrogen, Frozen canine or feline tissue, TRIzol® lysis reagent, Chloroform minimum 99%, Isopropyl Alcohol, 70% Ethanol (prepared in house with Ethanol, Absolute and deionized, RNase-free water), RNase Zap®, Deionized water, RNA Storage Solution®, from Ambion.

Equipment: Ultra-Turrax T25 Power Homogenizer, Beckman Coulter Allegra 25R Centrifuge, Eppendorf Centrifuge, Forceps, Scalpel, Hard cutting surface, i.e. cutting board, 1.5mL DNase and RNase free/sterile microcentrifuge tubes, 50mL DNase and RNase free/sterile disposable polypropylene tubes, P1000, P200, P20, P10 and P2 Rainin Pipetman pipettes, Filter pipette tips for P1000, P200, P20, P10 and P2 pipettes, DNase and RNase free/sterile, and lint free wipes.

Preparations: Prepare 50mL polypropylene tubes with 4mL TRIzol® (One tube for each tissue selected for RNA isolation).

Tissue Homogenization: Fill a container capable of holding liquid nitrogen with 3-4 scoops of liquid nitrogen. Place a piece of frozen tissue immediately into the aforementioned container (the tissue should be about the size of a pea) and place the tissue into the appropriate labeled 50mL polypropylene tube (that already contains 4mL TRIzol®). Immediately begin homogenization using the Ultra-Turrax T25 Power Homogenizer. Homogenize on the highest setting (6) for 10-15 seconds. Cool the sample on ice for another 10-15 seconds and then repeat. Continue until the tissue is fully homogenized and the solution is cloudy. Upon complete homogenization, cap the 50mL tube and return to the ice. Incubate the homogenized tissues at room temperature for 5 minutes before proceeding with the isolation procedure.

RNA Isolation: The procedures given in the Invitrogen instructions provided with the TRIzol® reagent are generally followed. Separate the homogenized sample into four 1mL aliquots in four 1.5mL microcentrifuge tubes. Add 200uL of chloroform to each 1mL aliquot. Cap the tubes, vortex for 15 seconds and then shake up and down. The result should be a pink milky liquid. Incubate the tubes at room temperature for 2-3 minutes. Centrifuge the tubes for 15 minutes at 14,000 rpm and 4°C. Transfer the aqueous phase (top layer) to a sterile 1.5mL microcentrifuge tube. The typical volume of the aqueous phase which should be transferred to the new tube is about 500uL. Be sure not to transfer any of the intermediate or lower phase. Precipitate the RNA from solution by adding 500uL of Isopropyl Alcohol to each microcentrifuge tube containing the aqueous layer. Shake the tubes up and down for at least 20 seconds. Incubate the samples at room temperature for 10 minutes. Centrifuge the samples for 10 minutes, 14,000 rpm at 4°C. Remove the supernatant carefully by aspirating off the liquid being sure not to lose the pellet. Add 1 mL of 70% ethanol to wash the pellet. Dislodge the pellet by flicking the tube (or tapping the tube on the bench top) and shake to mix. Centrifuge for 5 minutes, 8,200 rpm at 4°C. Remove the supernatant carefully by aspirating off the liquid being sure not to lose the pellet. Using a lint free wipe carefully soak up excess ethanol to make sure the pellet is dry. Resuspend each pellet into 30uL of RNA Storage Solution. Mix gently by pipetting until the RNA goes back into solution and then store at - 80°C. It may be necessary to vortex the sample for a few seconds at a low speed to facilitate the resuspension of the RNA. If this is necessary, spin down the samples, using the microcentrifuge, prior to freezing.

RNA Cleaning: The procedures given in the RNeasy® Mini Handbook are followed. RNA Isolation from Cells Cultured in OptiCell Chambers Using the RNeasy Mini Kit

Cells cultured from mammalian cell lines are used to isolate good quality RNA which is then used for future downstream genomic analysis. All work related to the culturing of the cells is to be done under strict aseptic conditions.

Reagents: 10X PBS, deionized H2O, Absolute ethanol, RNA Storage Solution, β-Mercaptoethanol, RNase Zap®, Buffer RLT, and Buffer RW1 and Buffer RPE (provided in the RNeasy Mini Kit)

Equipment/Materials: RNeasy Mini Kit, QIAshredder spin columns, OptiCell knife, 20mL sterile syringe, OptiCell tips, Cell scraper, P1000 Pipetman pipette, Rainin, P200 Pipetman pipette, Rainin, 100-100uL filtered pipette tips, 1-200uL filtered pipette tips, Sterile transfer pipettes, 55mL sterile solution basin, 1.5mL sterile microcentrifuge tubes, and Eppendorf Microcentrifuge.

Solutions: Buffer RLT (stock provided in RNeasy Mini Kit); -Add 100uL of β-Mercaptoethanol per 10mL of Buffer RLT prior to beginning protocol. 70% Ethanol: Make 50mL of 70% ethanol by adding 35mL absolute ethanol to 15mL deionized, RNase-free water. 1X PBS: RNase-free water. Filter the solution using a.22um filter

Procedure: Removing Cells from the OptiCell Chamber (proceed one OptiCell at a time). Check the cells under a microscope to ensure that the cells are alive before isolating RNA. Remove and discard the cell culture medium. Using the OptiCell knife cut away the top membrane exposing the cells on the lower membrane. Wash the membrane to which the cells are attached three times with 1X PBS. Pipette 600uL of the Buffer RLT solution (containing β-Mercaptoethanol) onto the center of the membrane to which the cells are attached. Using the cell scraper, gently spread the Buffer RLT over the entire surface of the membrane, and then collect the liquid in one corner. Pipette off the entire volume of Buffer RLT and place into a QIAshredder spin column.

RNA Isolation: Centrifuge the QIAshredder spin columns at 14,000 rpm for 2 minutes. Discard the spin column but keep the collection tube and its contents. Add 600uL of 70% ethanol to the collection tube and mix well by pipetting (the total volume now = 1.2mL). Transfer 600uL of the cell lysate to an RNeasy mini column and centrifuge for 15 seconds at 14,000 rpm. Discard the flow through but keep the collection tube and the spin column. Transfer the remaining volume of cell lysate (∼600uL) to the spin column and repeat the centrifugation. Discard the flow through but keep the collection tube and the spin column. Add 700uL Buffer RW1 to the spin column. Centrifuge for 15 seconds at 14,000 rpm to wash the column. Discard the flow through and the collection tube. Transfer the spin column to a new 2mL collection tube and add 500uL Buffer RPE to the column. Centrifuge for 15 seconds at 14,000 rpm. Discard the flow through, keep the collection tube/column. Add another 500uL Buffer RPE to the column. Centrifuge for 2 minutes at 14,000 rpm. Transfer the spin column to a 1.5mL collection tube. Add 30uL of RNA Storage Solution directly to the silica gel membrane and centrifuge for 1 minute at 14,000 rpm to elute the RNA. Store the final RNA at -70°C.

### RNA 6000 Nano Assay

Using the Agilent 2100 Bioanalyzer and the RNA 6000 Nano Assay, analyze RNA isolated from cultured mammalian cells, lymphocytes or tissues for quality.

Reagents: RNA 6000 Nano gel matrix, RNA 6000 Nano dye concentrate, RNA 6000 Nano Marker, (all of the above reagents are contained in the RNA 6000 Nano Assay kit, Agilent), RNA 6000 ladder , RNase Zap, and RNase-free water, from Ambion.

Equipment/Other Materials: Agilent Chip Priming Station, Agilent, RNA 6000 chip, Agilent, Electrode cleaners, P2, P10, P200, and P1000 Rainin Pipetman pipettes, Sterile, DNase/RNase free filtered pipette tips, 1.5mL microcentrifuge tubes, sterile, Vortex, IKA Vortex mixer, Microcentrifuge, and Heating block.

Procedure: The procedure is given in the Reagent Kit Guide, RNA 6000 Nano Assay, Edition November 2003, by Agilent Technologies. The procedures are followed as given in the Guide, with the following modifications: Preparing the Gel, pg. 17. Rather than separating the filtered gel into aliquots of 65uL each, keep the stock filtered gel in the original microcentrifuge tube and aliquot the 65uL as needed. Loading the RNA 6000 Nano Marker, pg. 22. Add 1uL of RNase-free water (instead of RNA 6000 Nano Marker) to each sample well that will not contain sample. Not only will this conserve the amount of Marker used but also serves as a negative control to see that none of the reagents are contaminated, including the RNase-free water. Loading the Ladder and Samples, pg. 23. Heat denature the samples and RNA 6000 Ladder for an additional 30 seconds (total of 2.5 minutes) at 71°C. Starting the Chip Run, pg. 26. Choose the "Eukaryote Total RNA Nano" option from the assay menu.

### Affymetrix Genechip Expression Analysis

Gene expression was analyzed using Affymetrix Canine 1 and Canine 2 GeneChip® Arrays available commercially from Affymetrix, Inc., Santa Clara, CA 95051. Total RNA is reverse transcribed into cDNA. The cDNA is used to generate cRNA which is fragmented and used as probes for GeneChip hybridization. The gene chip is washed and the hybridization signal is measured with an Affymetrix laser scanner. The hybridization data is then validated and normalized for further analysis.

Materials: Affymetrix provides most of the reagents and kit. Other reagents listed in the Affymetrix Manual but not supplied in the kit may be obtained separately. Refer to GeneChip Expression Analysis Technical Manual (701021 Rev.4) for the details. RNase Zap® and Deionized water.

Equipment: Eppendorf Microcentrifuge, 1.5mL DNase and RNase free/sterile microcentrifuge tubes, 504mL DNase and RNase free/sterile disposable polypropylene tubes, P1000, P200, P20, P10 and P2 Rainin Pipetman pipettes, Filter pipette tips for P1000, P200, P20, P10 and P2 pipettes, DNase and RNase free/sterile, and Peltier Thermal Cycler PTC-200.

Procedure: All procedures follow exactly as described in GeneChip Expression Analysis Technical Manual (Affymetrix Copyright 1999-2003). Use 5 microgram of total RNA for the first strand cDNA synthesis. Use either Peltier Thermal Cycler PTC-200 or heat block for temperature control on reactions and probe denaturing. The quality control is performed using RNA NanoDrop chips with BioAnalyer 2100. Use 100 Format (Midi Array) for the canine genechip.

### Example 1

### Determining the Effect of Various Substances or Ingredients on Gene Expression in Canine Cell Lines

Affymetrix canine gene chips Canine-1 and Canine-2 are used to determine the effect of various test substances or ingredients such as MCTs; TAGs; ALA; EPA; DHA; linoleic acid; stearic acid (SA), conjugated linoleic acid (CLA), GLA; arachidonic acid; lecithin; vitamin A, vitamin D, vitamin E, vitamin K, riboflavin, niacin, pyridoxine, pantothenic acid, folic acid, biotin vitamin C, catechin, quercetin, theaflavin; ubiquinone; lycopene, lycoxanthin; resveratrol; α-lipoic acid; L-carnitine; D-limonene; glucosamine; S-adenosylmethionine; chitosan, various materials containing one or more of these compounds, and various combination thereof on gene expression in four canine cell lines and appropriate controls. Each ingredient was tested in two concentrations as illustrated for selected sample ingredients shown in Table 6. The solvent at the higher of the two concentrations was used as a control. Four canine cell lines are used: CCL34 (kidney), CRL1430 (thymus), CCL183 (bone) (Obtained from The American Tissue Culture Collection) and CTAC (thyroid) (See, Measurement of NK Activity in Effector Cells Purified from Canine Peripheral Lymphocytes, Veterinary Immunology and Immunopathology, 35 (1993) 239-251). A cell line treated with an ingredient at a specific concentration is referred to as "treatment" and an untreated sample is referred to as "control." The words "genes" and "probes" are used synonymously in this method. Gene expression was measured for the treatment cell lines and controls.

The gene expression data was determined to be either "up" or "down" -regulated for any given treatment. The decision on whether a gene is "up" or "down" is based on the fold change, which is calculated as treatment intensity/control intensity for each individual probe. The fold change is considered down-regulated if its value is < 1/1.5 (for across all 4 cell lines analysis) or < 1/2 (for within cell lines analysis) and is up-regulated if it is > 1.5 (for across all 4 cell lines analysis) or > 2 (for within cell lines analysis). Also, a probe is considered significant for further scrutiny if it is called as present in only one of the conditions being compared (treatment or control) and is "absent" or "marginal" in the other and the fold change is significant according to the software used. Probes that appear to be regulated in opposite directions in the two treatments are excluded from further analysis.

The raw data is analyzed using GeneSpring version 7.0 (GS) software (Agilent Corporation) and validated using the R-Bioconductor (RB) freeware. Both software packages are used to compute probe intensities from the .CEL files generated by the Affymetrix Instrument. The Present/Absent/Marginal calls per probe and P-values are computed using the R-Bioconductor and GeneSpring software separately.

Two schemes are used for data analysis. First; "across cell lines" and "within individual cell lines." In the first scheme, genes are selected for scoring provided they are found to be significant and common across all cell-lines. The "across cell lines" yields the highest confidence data with minimum noise and may provide the best possible clues as to which genes are affected by individual ingredients. In the second scheme, only those genes that show a significant fold change in the two treatments according to both software packages within an individual cell lines are scored. A sample of the data obtained from these experiments is shown in Table 7. Table 7 shows the correlation between treatment substance (Column 1), Probe (data link) (Column 2), Direction (Column 3), Best BLAST Annotation (Column 4), and Human Accession Number of the closest human sequence to the target (Column 5). For clarity, the data shown in the table is only a small portion of the data obtained from the experiments conducted and is shown to illustrate the relevant aspects of the present invention, e.g., data indicates that the BDCs tested may affect "bottleneck" gene products that are central to fat metabolism including pyruvate dehydrogenase kinase and carnitine palmitoyl transferase I. The data also indicates that the ingredients that affect those two genes *in vitro* may be useful in doing the same when included in a diet that can subsequently be fed to fat dogs to enhance weight loss or fed to lean dogs to maintain leanness. The information for all ingredients tested is stored in a database for reference. This information comprises the second data set of the present invention.

### Example 2

### Determining Differential Gene Expression between Adipose Tissue Samples from Fat and Lean Animals

Adipose tissue samples are obtained from 13 fat and 3 lean canine animals diagnosed as either "fat" or "lean" using conventional methods. The "fatness" or "leanness" of an animal was determined based on measurements by DEXA using conventional methods or based on a 5 point body condition scoring system. For example, an animal was considered to be fat if it had a body condition score of 4 or higher and a total body fat percentage of 30% or higher. An animal was considered lean if it had a body condition score of 2 or 2.5 and/or a DEXA total body fat percentage of 27% or less. All tissue samples are snap frozen in liquid nitrogen immediately after removal from the animal.

The tissues are analyzed using Affymetrix "Canine- 2" canine gene chip according to conventional methods in order to determine which genes, if any, are differentially expressed in fat compared to lean animals. Data from the fat and lean samples are compared and analyzed using the GeneSpring and R-Bioconductor software. For any given gene to be assigned a "present" call it had to exhibit a 2-fold change in expression level to be considered for further scrutiny. Furthermore, genes that are present in only one condition and are either "absent" or "marginal" in the other group are also selected for further scrutiny.

A sample of data obtained using Example 2 is shown in Tables 3, 4, and 5. Table 3 shows an arbitrary SEQ ID NO in Column 1, the Affymetrix Probe Identification Number (herein "APIN") in Column 2, fold expression (fat/lean) in Column 3, Accession Number of Highest BLAST Hit in Column 4, and Accession Number of Highest BLAST Hit for a Human Sequence in Column 5. Table 4 shows the gene description obtained for the highest blast hit accession number for the corresponding SEQ ID NO and Table 5 shows the gene description for the highest blast hit for a human sequence accession number for the corresponding SEQ ID NO. For clarity, the data shown in the Tables is only a small portion of the data obtained from the experiments conducted and is shown to illustrate the relevant aspects of the present invention. In particular the data indicates that fat animals express the "bottleneck" gene products mentioned in example 1 at levels that are 2-3 times lower than in lean animals. Therefore, targeting the aforementioned bottleneck gene products using BDCs incorporated into a diet fed to fat animals may enhance their weight loss and help them maintain a lean profile. The information is stored in a database for reference. This information comprises the first data set of the present invention.

### Example 3

### Genes expressed differentially in the blood of fat and lean animals that can be used as class predictors for fat and lean animals.

In order to simplify our future tests and eliminate the need for using solid tissue samples that have to be biopsied from live animals, blood samples from fat and lean dogs are obtained and are used to develop a class predictor that can be used to differentiate between fat and lean animals. Affymetrix Canine-2 GeneChips are used to measure the gene expression levels in blood samples taken from animals that are identified as clinically fat (28 animals with a body condition score of 4 or 5) or lean (12 animals with a body condition score of 2 or 2.5). The GeneChip data is analyzed using the program GeneSpring (from Agilent Technologies) version 7.2. Sixty five probes that exhibit differential expression levels between the fat and lean samples with a "p" value of 0.01 after the application of a false discovery rate correction are identified. These probes (shown in table 8) and the genes and gene products that they represent can potentially be used as class predictors for fat and lean animals using blood samples without the need to use adipose tissue samples.

Based upon the physiological condition of the canines (a diagnosis as fat) and a comparison of the information from the data sets and Tables (the selection of the same genes that are influenced by a test substance or ingredient and are differentially expressed in fat compared to lean canines), a nutritional formula useful for selecting and preparing a food composition for fat canines is determined to contain one or more of the following ingredients in the following amounts (milligrams per kilogram of body weight per day (mg/kg/day): DHA - from about 1 to about 30; EPA - from about 1 to about 30; EPA/DHA Combo (1.5:1 ratio) - from about 4/2 to about 30/45; ALA - from about 10 to about 100; LA - from about 30 to about 600; ARA - from about 5 to about 50; and SA - from about 3 to about 60. Based upon this formula, a food composition and related diet containing one or more of these ingredients can be prepared and used to regulate the genes that are differentially expressed in fat compared to lean animals. Such regulation will cause the fat animal to modulate the amount of adipose tissue on the animal and, therefore, in one embodiment, promote a shift to a desirable or normal (more lean) status and promote better health and wellness of the animal.

### Example 4

### Diets containing higher amounts of long chain fatty acids promote weight loss and can be used to re-program the gene expression of the animal so that it reflects a propensity to become lean and potentially maintain leanness

The data obtained from in vitro ingredient screens discussed above indicate that some ingredients that are high in long chain fatty acids may have the potential to affect the expression of genes involved in fat metabolism in a way that would promote leanness of the animal as a whole. This is determined by analyzing data obtained from adipose tissue and from the ingredient assays discussed above using, e.g., conventional computer algorithm analyses. Code for algorithms useful in this regard are familiar to one of skill in the art and may be developed without undue experimentation. An example of such code is provided below:
SELECT A.PROBE, TO_CHAR(AVG(DECODE(A.EXPTDAY, 'D0', GENE_NORM_INT, null))/AVG(DECODE(A.EXPTDAY,'D14', GENE_NORM_INT, null)),'99999.99999') FATLEAN_FC, STATS_T_TEST_INDEPU(A.EXPTDAY, GENE_NORM_INT) P_VALUE, B.TOP_HIT_DEF,
COUNT(DISTINCT C.INGREDIENT),
COUNT(DISTINCT D.INGREDIENT)
FROM GERIATRICS_RNRM2 A, TOP_PROBE_ANNOT_2_3 B,
FILT_INDIV_CELLS_2 C, FILT_ACROSS_4_CELLS_2 D
WHERE A.PROBE=B.PROBE AND A.PROBE=C.PROBE (+) AND
A.PROBE=D.PROBE (+) AND
UPPER(A.PROBE) NOT LIKE 'AFFX%'
GROUP BY A.PROBE, B.TOP_HIT_DEF
HAVING STATS_T_TEST_INDEPU(A.EXPTDAY, GENE_NORM_INT) <= .01 AND
AVG(DECODE(A.EXPTDAY, 'D0', GENE_NORM_INT, null))/AVG(DECODE(A.EXPTDAY, 'D14', GENE_NORM_INT, null)) >= 5 AND
SUM(DECODE(PAMCALL, 'P', 1, 0)) = 40 ORDER BY PROBE

To confirm that the inclusion of linolenic acid or EPA/DHA (1.5:1) in diets fed to dogs does affect weight loss in dogs, three high protein diets containing either no added long chain fatty acids (Diet A) or added linolenic acid (approximately 1% based on 100% dry matter basis, Diet B) or EPA/DHA (1.5:1, approximately 0.30%: 0.20 %) (Diet C) were developed for comparison to a high fiber diet that is known to induce weight loss in dogs. In the study, 45 clinically fat dogs are all first fed a nutritionally complete control diet for 30 days prior to the start of the test. After the initial 30 days, the dogs are randomized into 4 groups. Three of the four groups receive one of the test diets and one group is given the high fiber diet as a control for a set period of time, e.g., 4 months.. Results indicate that the three experimental foods (Diets A, B and C) have substantially higher digestibility than the higher fiber food. Results also indicate that approximately 38% of the dogs consuming the food containing EPA/DHA reach their weight loss goal at 90 days. Interestingly, dogs consuming the EPA/DHA food also maintain lean muscle mass and bone mineral content. The results also indicate that, at least at the clinical level, diets containing EPA/DHA may be as effective as high fiber diets in affecting weight loss.

In order to validate the class predictor probe set and to test its ability to predict fatness or leanness in animals, the class predictor probe set (described in Example 3 above) is applied to gene expression data obtained from the 45 animals participating in the experiment above (expression data not shown). The class predictor analysis confirms that 41 of the 45 animals (approximately 90%) designated "fat" at the beginning of the test are in fact fat (the discrepancy may be due to the subjective nature of the conventional body condition scoring system that is currently used in the clinic). Interestingly, after 14 days of feeding the four diets described above, the class predictor analysis indicates that all animals, regardless of diet, display a "lean" gene expression profile. At the end of the study, it appears that all the animals on the control high fiber diet reflect a "fat" gene expression profile, approximately 25% of the animals on test Diets A and B reflect a biochemically "lean" gene expression profile and approximately 40% of the animals fed on Diet C containing EPA/DHA exhibit a biochemically "lean" gene expression profile. (see Table 9).

### TABLES

**Table 1**

| Specimen Nutritional Formula | | |
|---|---|---|
| Formula for (identifier of animal): | | Spot |
| Species: | | Canine |
| Breed: | | Dalmatian |
| Age: | | 5 yr 3 mo |
| FGP identifier: | | [code enabling retrieval of FGP data] |
| Base | | |
| | Energy density (kcal ME/g) | 3.5-4.5 |
| | Crude protein (%) | 15-30 |
| | Crude fat (%) | 10-20 |
| | Crude fiber (%) | 2-5 |
| | Calcium (%) | 0.5-1.0 |
| | Phosphorus (%) | 0.4-0.9 |
| | Sodium (%) | 0.2-0.4 |
| | Chloride (%) | 0.3-0.6 |
| BDC supplementation | | |
| | Vitamin A (IU/g) | 100 |
| | EPA (mg/g) | 0.25 |
| | S-adenosylmethionine (mg/g) | 0.2 |
| | Powdered ginger (mg/g) | 5 |

**Table 2**

| Specimen Food Composition | |
|---|---|
| Food composition for (identifier of animal): | Fluffy |
| Species: | Feline |
| Breed: | Mixed |
| Age: | 2 yr 10 mo |
| Nutritional formula identifier: | [code enabling retrieval of nutritional formula] |
| Type of food: | Kibble |
| Chicken meal, low fat (%) | 50 |
| Corn, ground (%) | 23 |
| Soybean meal (%) | 10 |
| Fish meal (%) | 10 |
| Fish oil (%) | 3 |
| Dicalcium phosphate (%) | 0.3 |
| Evening primrose oil (%) | 0.05 |

**Table 3**

| Column | 1 | 2 | 3 | 4 | 5 |
|---|---|---|---|---|---|
| | 15 | CfaAffx.4097.1.S1_s_at | 0.32 | XM_539427 | BC040239 |
| | 62 | CfaAffx.16813.1.S1_at | 0.37 | XM_533208 | NM_001876 |
| | 67 | Cfa.101.1.S1_s_at | 0.35 | XM_533208 | BC000185 |
| | 70 | CfaAffx.22979.1.S1_s_at | 0.34 | XM_533208 | AJ420748 |
| | 241 | Cfa.2282.1.S1_at | 0.45 | XM_539427 | AK096428 |
| | 285 | Cfa.1286.1.A1_at | 0.42 | XM_583309 | CR599853 |

**Table 4**

| SEQ ID NO Gene Description - Highest BLAST Hit Accession Number | |
|---|---|
| 15 | PREDICTED: Canis familiaris similar to [Pyruvate dehydrogenase [lipoamide]] kinase isozyme 4, mitochondrial precursor (Pyruvate dehydrogenase kinase isoform 4) (LOC482310), mRNA |
| 62 | PREDICTED: Canis familiaris carnitine palmitoyl transferase I isoform (CPT1), mRNA |
| 67 | PREDICTED: Canis familiaris carnitine palmitoyl transferase I isoform (CPT1), mRNA |
| 70 | PREDICTED: Canis familiaris carnitine palmitoyl transferase I isoform (CPT1), mRNA |
| 241 | PREDICTED: Canis familiaris similar to [Pyruvate dehydrogenase [lipoamide]] kinase isozyme 4, mitochondrial precursor (Pyruvate dehydrogenase kinase isoform 4) (LOC482310), mRNA |
| 285 | PREDICTED: Bos taurus similar to Carnitine O-palmitoyltransferase I, mitochondrial liver isoform (CPT I) (CPTI-L) (Carnitine palmitoyltransferase 1A) (LOC506812), partial mRNA |

**Table 5**

| SEQ ID NO Gene Description - Highest BLAST Hit for a Human Sequence Accession Number | |
|---|---|
| 15 | Homo sapiens pyruvate dehydrogenase kinase, isozyme 4, mRNA (cDNA clone MGC:5281 IMAGE:3047987), complete cds |
| 62 | Homo sapiens carnitine palmitoyltransferase 1A (liver) (CPT1A), nuclear gene encoding mitochondrial protein, transcript variant 1, mRNA |
| 67 | Homo sapiens carnitine palmitoyltransferase 1A (liver), transcript variant 2, mRNA (cDNA clone MGC:1772 IMAGE:3352642), complete cds |
| 70 | Homo sapiens partial CPT1A gene for carnitine O-palmitoyltransferase 1, promoter region, CDS and slice variants a and b |
| 241 | Homo sapiens cDNA FLJ39109 fis, clone NTONG2005137, highly similar to [PYRUVATE DEHYDROGENASE(LIPOAMIDE)] KINASE ISOZYME 4, MITOCHONDRIAL PRECURSOR (EC 2.7.1.99) |
| 285 | full-length cDNA clone CSODK009YI05 of HeLa cells Cot 25-normalized of Homo sapiens (human) |

**Table 6**

| Substance | Concentration 1 | Concentration 2 | Solvent |
|---|---|---|---|
| DHA | 0.005 mg/ml (5 micro g/ml) | 0.025 mg/ml (25 micro g/ml) | ETOH |
| EPA | 0.005 mg/ml (5 micro g/ml) | 0.025 mg/ml (25 micro g/ml) | ETOH |
| EPA/DHA | 0.062 mg/ml EPA & 0.010 | 0.030 mg/ml EPA & 0.02 | ETOH |
| Combo 1.5:1 | mg/ml DHA (total is 0.025 | mg/ml DHA (total is 0.050 | |
| ratio (like in | mg/ml) | mg/ml) | |
| fish oil) | | | |
| Alpha linolenic | 0.05 mg/ml (50 micro g/ml) | 0.1 mg/ml (100 micro g/ml) | ETOH |
| acid | | | |
| Linoleic acid | 0.1 mg/ml (100 micro g/ml) | 0.5 mg/ml (500 micro g/ml) | ETOH |
| Arachidonic | 0.025 mg/ml (25 micro | 0.05 mg/ml (50 micro g/ml) | ETOH |
| acid | g/ml) | | |
| Stearic acid | 0.01mg/ml (10 micro g/ml) | 0.05 mg/ml (50 micro g/ml) | ETOH |
| Conjugated | 0.02 mg/ml (20 micro g/ml) | 0.1 mg/ml (100 micro g/ml) | MEOH |
| Linoleic acid | | | |

**Table 7**

| Column 1 | 2 | 3 | 4 | | 5 |
|---|---|---|---|---|---|
| DHA | 6282824_at | | UP | PREDICTED: Canis familiaris carnitine palmitoyl transferase I isoform (CPT1), mRNA | BC000185 |
| DHA | 1605486_at | | UP dehydrogenase | Homo sapiens pyruvate kinase 4 mRNA, 3' untranslated region, partial sequence | AK096428 |
| DHA | 6287734_at | | UP | PREDICTED: Canis familiaris similar to NalPi cotransporter 4 (LOC478741), mRNA | BC017952 |
| EPA | 6283329_at | | DOWN | Homo sapiens, Similar to | AC018634 |
| | | | | secreted frizzled-related protein 4, clone IMAGE:4828181, mRNA | |
| EPA | 6283403_at | | UP | Sus scrofa carnitine palmitoyltransferase I mRNA, nuclear gene encoding mitochondrial protein, complete cds | AK172798 |
| EPA | 1605486_at | | UP | Homo sapiens pyruvate dehydrogenase kinase 4 mRNA, 3' untranslated region, partial sequence | AK096428 |
| DHA/EPA | 6283403_at | | UP | Sus scrofa carnitine palmitoyltransferase I mRNA, nuclear gene encoding mitochondrial protein, complete cds | AK172798 |
| DHA/EPA | 1605486_at | | UP | Homo sapiens pyruvate dehydrogenase kinase 4 mRNA, 3' untranslated region, partial sequence | AK096428 |
| DHA/EPA | 1605832_at | | DOWN | Homo sapiens mRNA; cDNA DKFZp451J622 (from clone DKFZp451 J622); complete cds | AK097112 |
| ALA | 6282455_at | | DOWN | Canis familiaris type I collagen pre-pro-alphal (I) chain (COL1A1) mRNA, complete cds | AB209597 |
| ALA | 1605486_at | | UP | Homo sapiens pyruvate dehydrogenase kinase 4 mRNA, 3' untranslated region, partial sequence | AK096428 |
| LA | 6282385_at | | DOWN | Canis familiaris Na+- dependent glutamate transporter (GLAST), mRNA | D26443 |
| LA | 6282824_at | | UP | PREDICTED: Canis familiaris carnitine palmitoyl transferase I isoform (CPT1), mRNA | BC000185 |
| LA | 1605486_at | | UP | Homo sapiens pyruvate dehydrogenase kinase 4 mRNA, 3' untranslated region, partial sequence | AK096428 |
| ARA | 6282824_at | | UP | PREDICTED: Canis familiaris carnitine palmitoyl transferase I isoform (CPT1), mRNA | BC000185 |
| ARA | 6283403_at | | UP | Sus scrofa carnitine palmitoyltransferase I | AK172798 |
| | | | | mRNA, nuclear gene encoding mitochondrial protein, complete cds | |
| ARA | 1602749_at | | UP | Homo sapiens BAC clone RP11-44D21 from 4, complete sequence | AC108866 |
| ARA | 1605486_at | | UP dehydrogenase | Homo sapiens pyruvate kinase 4 mRNA, 3' untranslated region, partial sequence | AK096428 |
| SA | Cfa.2282.1.S1_at | | UP | PREDICTED: Canis familiaris similar to [Pyruvate dehydrogenase [lipoamide]] kinase isozyme 4, mitochondrial precursor (Pyruvate dehydrogenase kinase isoform 4) (LOC482310), mRNA | AK096428 |
| SA | Cfa.791.4.A1_at | | UP | PREDICTED: Canis familiaris similar to ribosomal protein L24, transcript variant 2 (LOC478547), mRNA | NM_000986 |
| SA | CfaAffx.9845.1.S1_s_at | | UP | PREDICTED: Canis familiaris similar to leucine rich repeat containing 45 (LOC483375), mRNA | NM_144999 |
| CLA | Cfa.10478.1.A1_at | | UP | PREDICTED: Bos taurus similar to Type II inositol-1,4,5-trisphosphate 5-phosphatase precursor (Phosphoinositide 5-phosphatase) (5PTase) (75 kDa inositol polyphosphate-5-phosphatase) (LOC538291), partial mRNA | AC005691 |
| CLA | Cfa.11267.1.A1_at | | DOWN | Homo sapiens cDNA clone IMAGE:4456146, partial cds | BC024645 |
| CLA | Cfa.11358.1.A1_at | | UP | Homo sapiens solute carrier family 20 (phosphate transporter), member 2 (SLC20A2), mRNA | AF170802 |

**Table 8: Affymetrix probes representing genes that can be used as class predictors for fat and lean animals using blood samples instead of adipose tissue samples**

| | Affymetrix | |
|---|---|---|
| | probe id | top-annotation based on BLAST sequence similarity PREDICTED: Canis familiaris similar to alpha-synuclein isoform |
| 1 | Cfa.10128.1.A1_at | NACP140; transcript variant 3 (LOC478478); mRNA PREDICTED: Canis familiaris similar to ADP-ribosylation |
| 2 | Cfa. 10772.1.A1_at | factor GTPase activating protein 3; transcript variant 5 (LOC474477); mRNA |
| 3 | Cfa.11444.1.A1 _at | Homo sapiens elk1 oncogene; complete cds PREDICTED: Canis familiaris similar to ubiquitin |
| 4 | Cfa.1152.1.A1_s_at | C-terminal hydrolase UCH37 (LOC478958); mRNA |
| 5 | Cfa.11624.1.A1_at | PREDICTED: Canis familiaris similar to retinaldehyde binding protein 1 (LOC479039); mRNA |
| | | PREDICTED: Canis familiaris similar to Coiled-coil-helix-coiled-coil-helix domain containing |
| 6 | Cfa.13515,1.S1_at | protein 3; transcript variant 5 (LOC607574); mRNA |
| 7 | Cfa.13669.1.A1_at | No available annotation |
| 8 | Cfa.15521.1.A1_at | Pongo pygmaeus mRNA; cDNA DKFZp468H0312 (from clone DKFZp468H0312) PREDICTED: Canis familiaris similar to NADH dehydrogenase (ubiquinone) 1 alpha |
| 9 | Cfa.16699.1.S1_s_at | subcomplex; 11; 14.7kDa; transcript variant 1 (LOC476735); mRNA PREDICTED: Canis familiaris similar to ADP-ribosylation factor GTPase activating protein 3; |
| 10 | Cfa.17093.1.S1_at | transcript variant 2 (LOC474477); mRNA |
| 11 | Cfa.18024.1.S1_s_at | PREDICTED: Canis familiaris similar to MAK31-like protein (LOC479488); mRNA |
| 12 | Cfa.1945.1.A1_at | No available annotation PREDICTED: Rattus norvegicus similar to hypothetical protein FLJ25439 (LOC502510); |
| 13 | Cfa.19577.1.S1_at | mRNA PREDICTED: Canis familiaris similar to NADH dehydrogenase (ubiquinone) 1 beta |
| 14 | Cfa.273.3.A1_s_at | subcomplex 8; transcript variant 1 (LOC477798); mRNA |
| 15 | Cfa.3698.1.A1_at | Canis familiaris angiotensin II type 2 receptor mRNA; partial cds |
| 16 | Cfa.3895.1.A1_s_at | Canis familiaris Sec61 beta subunit (Sec61 b); mRNA PREDICTED: Canis familiaris similar to NADH dehydrogenase (ubiquinone) Fe-S protein 6; |
| 17 | Cfa.4245.1.S1_s_at | 13kDa (NADH-coenzyme Q reductase) (LOC478629); mRNA |
| 18 | Cfa.4779.1.A1_at | PREDICTED: Bos taurus similar to mal; T-cell differentiation protein-like (LOC512289); mRNA |
| 19 | Cfa.5440.1.A1_at | Magnaporthe grisea 70-15 hypothetical protein (MG04641.4) partial mRNA PREDICTED: Canis familiaris similar to growth differentiation factor 3 precursor (LOC477702); |
| 20 | Cfa.5628.1.A1_s_at | mRNA PREDICTED: Canis familiaris similar to glyceraldehyde-3-phosphate dehydrogenase |
| 21 | Cfa.5672.1.A1_s_at | (LOC481027); mRNA |
| 22 | Cfa.583.1.S1_at | Homo sapiens mRNA; cDNA DKFZp761 M0111 (from clone DKFZp761 M0111) |
| 23 | Cfa.6307.1.A1_s_at | PREDICTED: Canis familiaris similar to presenilin enhancer 2 (LOC476479); mRNA |
| 24 | Cfa.6307.1.A1_x_at | PREDICTED: Canis familiaris similar to presenilin enhancer 2 (LOC476479); mRNA PREDICTED: Canis familiaris similar to adiponectin receptor 2; transcript variant 2 |
| 25 | Cfa.7730.1.A1_at | (LOC477732); mRNA PREDICTED: Canis familiaris similar to Kelch repeat and BTB domain containing protein 10 (Kelch-related protein 1) (Kel-like protein 23) (Sarcosin); transcript variant 3 (LOC478784); |
| 26 | Cfa.8497.1.A1_at | mRNA PREDICTED: Canis familiaris similar to MADS box transcription enhancer factor 2; |
| 27 | Cfa.9073.1.A1_s_at | polypeptide C (myocyte enhancer factor 2C); transcript variant 30 (LOC479155); mRNA |
| 28 | Cfa.9519.1.A1_at | full-length cDNA clone CSODF038YH13 of Fetal brain of Homo sapiens (human) PREDICTED: Canis familiaris similar to solute carrier family 5 (iodide transporter); member 8 |
| 29 | CfaAffx.11304.1.S1_at | (LOC482626); mRNA |
| 30 | CfaAffx.12600.1.S1_s_at | C.familiaris mRNA for TRAM-protein |
| 31 | CfaAffx.12899.1.S1_at | PREDICTED: Bos taurus similar to olfactory receptor Olr535 (LOC510433); mRNA |
| 32 | CfaAffx.13068.1.S1_s_at | Canis familiaris carboxypeptidase B1 (tissue) (CPB1); mRNA |
| 33 | CfaAffx.13084.1.S1_at | Mus musculus olfactory receptor MOR232-2 gene; complete cds |
| 34 | CfaAffx.13369.1.S1_s_at | PREDICTED: Canis familiaris similar to selenoprotein T (LOC612992); mRNA |
| 35 | CfaAffx.13927.1.S1_at | PREDICTED: Canis familiaris similar to CG10510-PA (LOC477622); mRNA PREDICTED: Canis familiaris similar to Transmembrane 9 superfamily protein member 3 |
| 36 | CfaAffx.13999.1.S1_s_at | precursor; transcript variant 5 (LOC612786); mRNA PREDICTED: Canis familiaris similar to chromodomain helicase DNA binding protein 6; |
| 37 | CfaAffx.14593.1.S1_s_at | transcript variant 1 (LOC477230); mRNA |
| 38 | CfaAffx.16220.1.S1_s_at | PREDICTED: Canis familiaris similar to membrane-spanning 4-domains; subfamily A; member 6A isoform 2 (LOC612553); mRNA |
| 39 | CfaAffx.16368.1.S1_s_at | Canine mRNA for signal recognition particle receptor PREDICTED: Canis familiaris similar to ubiquitin-conjugating enzyme E2G 2 (LOC611581); |
| 40 | CfaAffx.17233.1.S1_s_at | mRNA |
| 41 | CfaAffx.18688.1.S1_at | PREDICTED: Canis familiaris hypothetical protein LOC609372 (LOC609372); mRNA |
| 42 | CfaAffx.19132.1.S1_s_at | PREDICTED: Canis familiaris similar to uroplakin 2 (LOC610673); mRNA |
| 43 | CfaAffx.19769.1.S1_at | PREDICTED: Canis familiaris similar to YTH domain protein 1 (Dermatomyositis associated with cancer putative autoantigen-1 homolog) (DACA-1 homolog) (LOC485968); mRNA PREDICTED: Canis familiaris similar to patched domain containing 1; transcript variant 1 |
| 44 | CfaAffx.20665.1.S1_at | (LOC491775); mRNA |
| 45 | CfaAffx.20740.1.S1_s_at | PREDICTED: Canis familiaris similar to a disintegrin and metalloproteinase domain 23 preproprotein; transcript variant 2 (LOC607871); mRNA PREDICTED: Canis familiaris similar to Ferritin light chain (Ferritin L subunit) (LOC491829); |
| 46 | CfaAffx.21676.1.S1_at | mRNA PREDICTED: Canis familiaris similar to ADP-ribosylation factor GTPase activating protein 3; |
| 47 | CfaAffx.2327.1.S1_s_at | transcript variant 5 (LOC474477); mRNA |
| 48 | CfaAffx.23835.1.S1_at | Homo sapiens protocadherin 15 (PCDH15); mRNA PREDICTED: Canis familiaris similar to Growth hormone inducible transmembrane protein |
| 49 | CfaAffx.24356.1.S1_s_at | (Dermal papilla derived protein 2); transcript variant 3 (LOC479266); mRNA PREDICTED: Canis familiaris similar to Olfactory receptor 7A5 (Olfactory receptor TPCR92) |
| 50 | CfaAffx.24849.1.S1_at | (LOC610545); mRNA PREDICTED: Canis familiaris similar to Renal sodium-dependent phosphate transport protein 2 (Sodium/phosphate cotransporter 2) (Na(+)/Pi cotransporter 2) (Renal sodium-phosphate |
| 51 | CfaAffx.25142.1.S1_s_at | transport protein 2) (Renal Na(+)-dependent phosphate cotransporter 2); t Macaca fascicularis brain cDNA; clone:QfIA-12135; similar to human progestin and adipoQ |
| 52 | CfaAffx.25751.1.S1_at | receptor family member VI (PAQR6); mRNA; NM_024897.2 |
| 53 | CfaAffx.26483.1.S1_s at | Canis familiaris non-metastatic cells 2; protein (NM23B) expressed in (NME2); mRNA PREDICTED: Canis familiaris similar to CD27-binding (Siva) protein isoform 1 (LOC612693); |
| 54 | CfaAffx.28078.1.S1_s_at | mRNA PREDICTED: Canis familiaris similar to Ubiquitin-conjugating enzyme E2 A (Ubiquitin-protein |
| 55 | CfaAffx.28164.1.S1_at | ligase A) (Ubiquitin carrier protein A) (HR6A) (mHR6A) (LOC492095); mRNA PREDICTED: Canis familiaris similar to Coiled-coil-helix-coiled-coil-helix domain containing |
| 56 | CfaAffx.2860.1.S1_s_at | protein 3; transcript variant 2 (LOC607574); mRNA |
| 57 | CfaAffx.28798.1.S1_at | PREDICTED: Canis familiaris similar to seizure related gene 6 (LOC491175); mRNA |
| 58 | CfaAffx.29250.1.S1_s_at | PREDICTED: Canis familiaris similar to CG4646-PA (LOC479563); mRNA |
| 59 | CfaAffx.32063.1.S1_at | No available annotation PREDICTED: Canis familiaris similar to ADAM DEC1 precursor (A disintegrin and metalloproteinase domain-like protein decysin 1) (ADAM-like protein decysin 1) (LOC608742); |
| 60 | CfaAffx.360.1.S1_s_at | mRNA |
| 61 | CfaAffx.3860.1.S1_s_at | Homo sapiens mRNA for KIAA1045 protein; partial cds PREDICTED: Canis familiaris similar to zinc finger protein 91 (HPF7; HTF10) (LOC484590); |
| 62 | CfaAffx.604.1.S1_at | mRNA PREDICTED: Canis familiaris similar to progesterone membrane binding protein |
| 63 | CfaAffx.6669.1.S1_at | (LOC476084); mRNA |
| 64 | CfaAffx.7079.1.S1_at | PREDICTED: Canis familiaris TATA-box binding protein (LOC475040); mRNA PREDICTED: Canis familiaris similar to mitochondrial ribosomal protein L48 isoform 1 |
| 65 | CfaAffx.9326.1.S1_s_at | (LOC476812); mRNA |

**Table 9: Percent of Lean Animals as Predicted by the 65-probe Class Predictor**

| | **Diet Day 0** | **Diet Day 14** | **Diet Day 120** |
|---|---|---|---|
| **Diet A (n=12)** | 9% | 33% | 25% |
| **Diet B (n=10)** | 10% | 40% | 25% |
| **Diet C (n=14)** | 7% | 29% | 40% |
| **High Fiber Diet (n=9)** | 11% | 30% | 0% |

Aspects of the invention are described with reference to the following clauses:
1. A method of selecting a food composition for an animal subpopulation comprising:
   (a) accessing at least one database that comprises a first data set relating a functional genomic profile of a biofluid or tissue sample from an animal to physiological condition and optionally genotype of the animal;
   (b) accessing at least one database that comprises a second data set relating to effects of bioactive dietary components on functional genomic profile; and
   (c) by use of a first algorithm drawing on the first and second data sets, processing input data defining physiological condition and optionally genotype of the subpopulation to derive a nutritional formula useful for selecting and preparing a food composition for an animal subpopulation.
2. The method of Clause 1 further comprising preparing a food composition based on the nutritional formula.
3. The method of Clause 1 wherein the subpopulation comprises a companion animal.
4. The method of Clause 1 wherein the subpopulation is selected from the group consisting of canine and feline.
5. The method of Clause 1 wherein the subpopulation is defined at least in part by a characteristic selected from the group consisting of one or more of breed type, specific breed, chronological age, physiological age, activity level, state of wellness, and state of disease.
6. The method of Clause 1 wherein the first data set is derived from samples collected from a multiplicity of individual animals representative of a range of genotypes and physiological conditions that includes the subpopulation, each such sample from an individual animal being associated with a provenance record that comprises zoographical data relevant to defining the genotype and physiological condition, at the time the sample is collected, of the individual animal.
7. The method of Clause 6 wherein the zoographical data comprise one or more data items relating to genotype, selected from the group consisting of breed, breed(s) of parents, pedigree, sex, coat type, and evident hereditary conditions and disorders.
8. The method of Clause 6 wherein the zoographical data comprise one or more data items relating to physiological condition, selected from the group consisting of age, weight, veterinary medical history, reproductive history, present wellness or disease state, appetite, physical activity level, mental acuity, behavioral abnormalities and disposition.
9. The method of Clause 1 wherein the first data set comprises data relating to analysis of the sample with respect to one or more components selected from the group consisting of DNA, RNA, proteins, metabolites and biomarkers.
10. The method of Clause 1 wherein the second data set is derived from controlled experiments comprising exposing an animal model to different levels of one or more bioactive dietary components.
11. The method of Clause 1 wherein the input data comprise zoographical data relevant to defining the genotype and physiological condition of the subpopulation.
12. The method of Clause 1 wherein the input data comprise analytical data from a biofluid or tissue sample obtained from an animal of the subpopulation.
13. The method of Clause 12 wherein the analytical data relate to one or more components selected from the group consisting of DNA, RNA, proteins, metabolites and biomarkers.
14. The method of Clause 12 wherein the analytical data comprise a functional genomic profile of the animal.
15. The method of Clause 1 wherein the nutritional formula promotes wellness by enhancing an aspect of health of one or more animals of the subpopulation.
16. The method of Clause 1 wherein the nutritional formula promotes wellness by preventing, attenuating or eliminating at least one disease state in one or more animals of the subpopulation.
17. The method of Clause 16 wherein a cluster of two or more disease states are simultaneously prevented, attenuated or eliminated.
18. A food composition prepared by the method of Clause 1.
19. A method of diagnosing a state of wellness, disease or physiological disorder, or a predisposition to disease or physiological disorder in an animal subject comprising:
   (a) accessing at least one database that comprises a sample data set from which normal and extranormal functional genomic profiles can be identified for animals having ranges of genotype and physiological condition that encompass the genotype and physiological condition respectively of the subject; and
   (b) by use of an algorithm drawing on the test data set, processing input data that define a functional genomic profile for the subject to derive a diagnosis.
20. The method of Clause 19 further comprising prescribing a treatment or prophylaxis for the subject based on the diagnosis.
21. A method for predicting the "physiological" class or condition of an animal and its propensity to develop disease or to respond to a given nutritional treatment comprising:
   a) applying supervised learning algothrims to genomic, proteomic and/or metabolomic data obtained from a learning set of animals or samples that exhibit different physiological states;
   b) determining a class prediction rule;
   c) applying the class prediction rule to a new set of test samples;
   d) classifying or assigning membership of the test samples and therefore the animal that provided the sample, to a particular physiological state based on the class prediction outcome resulting from step (c); and
   e) using the results of step (d) to determine means for bringing an animal from an abnormal physiological state to a normal physiological state using BDCs incorporated into diets.
22. The method of Clause 21 further including an additional step (f) comprising using the class prediction rule to follow the animal's response to the treatment described in step (e) of said method.

## Claims

1. A method of selecting a food composition for an animal subpopulation executed by a computer-aided system comprising:
(a) accessing at least one database that comprises a first data set relating a functional genomic profile of a biofluid or tissue sample from an animal to physiological condition and genotype of the animal;
(b) accessing at least one database that comprises a second data set relating to effects of bioactive dietary components on functional genomic profile; and
(c) by use of a first algorithm drawing on the first and second data sets, processing input data defining physiological condition and genotype of the subpopulation to derive a nutritional formula useful for selecting and preparing a food composition for the animal subpopulation, wherein the bioactive dietary components comprise amino acids; simple sugars; complex sugars; medium-chain triglycerides (MCTs); triacylglycerides (TAGs); n-3 (omega-3) fatty acids; n-6 (omega-6) fatty acids; choline sources; fat-soluble vitamins; water-soluble vitamins; antioxidants; bioflavonoids; quinones; carotenoids; resveratrol; α-lipoic acid; L-carnitine; D-limonene; glucosamine; S-adenosylmethionine; and/or chitosan.

2. The method of claim 1, wherein the n-3 (omega-3) fatty acids comprise α-linolenic acid (ALA), eicosapentaenoic acid (EPA) or docosahexaenoic acid (DHA); the n-6 (omega-6) fatty acids comprise linoleic acid (LA), γ-linolenic acid (GLA) or arachidonic acid (ARA); the choline sources comprise lecithin; the fat-soluble vitamins comprise vitamin A and precursors thereof such as carotenoids (*e.g.*, β-carotene), vitamin D sources such as vitamin D₂ (ergocalciferol) and vitamin D₃ (cholecalciferol), vitamin E sources such as tocopherols (*e.g*., α-tocopherol) and tocotrienols, or vitamin K sources such as vitamin K₁ (phylloquinone) and vitamin K₂ (menadione); the water-soluble vitamins comprise B vitamins such as riboflavin, niacin (including nicotinamide and nicotinic acid), pyridoxine, pantothenic acid, folic acid, biotin and cobalamin; or vitamin C (ascorbic acid); the antioxidants comprise vitamins E or C; the bioflavonoids comprise catechin, quercetin or theaflavin; the quinones comprise ubiquinone; and/or the carotenoids comprise lycopene or lycoxanthin.

3. The method of claim 1, wherein the bioactive dietary components comprise medium-chain triglycerides (MCTs); triacylglycerides (TAGs); α-linolenic acid (ALA), eicosapentaenoic acid (EPA), docosahexaenoic acid (DHA); linoleic acid (LA), stearic acid (SA), conjugated linoleic acid (CLA), γ-linolenic acid (GLA), arachidonic acid (ARA); lecithin; vitamin A, vitamin D, vitamin E, vitamin K, riboflavin, niacin, pyridoxine, pantothenic acid, folic acid, biotin, vitamin C (ascorbic acid); catechin, quercetin, theaflavin; ubiquinone; lycopene, lycoxanthin; resveratrol; α-lipoic acid; L-carnitine; D-limonene; glucosamine; S-adenosylmethionine; and/or chitosan.

4. The method of Claim 1 further comprising preparing a food composition based on the nutritional formula.

5. The method of Claim 1, wherein
the subpopulation comprises a companion animal, or
the subpopulation is selected from the group consisting of canine and feline, or the subpopulation is defined by a characteristic selected from the group consisting of one or more of breed type, specific breed, chronological age, physiological age, activity level, state of wellness, and state of disease.

6. The method of Claim 1, wherein
the first data set is derived from samples collected from a multiplicity of individual animals representative of a range of genotypes and physiological conditions that includes the subpopulation, each such sample from an individual animal being associated with a provenance record that comprises zoographical data relevant to defining the genotype and physiological condition, at the time the sample is collected, of the individual animal, or
the first data set comprises data relating to analysis of the sample with respect to one or more components selected from the group consisting of DNA, RNA, proteins, metabolites and biomarkers.

7. The method of Claim 6, wherein
the zoographical data comprise one or more data items relating to genotype, selected from the group consisting of breed, breed(s) of parents, pedigree, sex, coat type, and evident hereditary conditions and disorders, or
the zoographical data comprise one or more data items relating to physiological condition, selected from the group consisting of age, weight, veterinary medical history, reproductive history, present wellness or disease state, appetite, physical activity level, mental acuity, behavioral abnormalities and disposition.

8. The method of Claim 1 wherein the second data set is derived from controlled experiments comprising exposing an animal model to different levels of one or more bioactive dietary components.

9. The method of Claim 1, wherein the input data comprise
zoographical data relevant to defining the genotype and physiological condition of the subpopulation, or
analytical data from a biofluid or tissue sample obtained from an animal of the subpopulation and optionally, wherein:
(i) the analytical data relate to one or more components selected from the group consisting of DNA, RNA, proteins, metabolites and biomarkers, or
(ii) the analytical data comprise a functional genomic profile of the animal.

10. The method of Claim 1 wherein the nutritional formula promotes wellness by enhancing an aspect of health of one or more animals of the subpopulation.

11. The method of Claim 1 wherein the nutritional formula promotes wellness by preventing, attenuating or eliminating at least one disease state in one or more animals of the subpopulation.

12. The method of Claim 11 wherein two or more disease states are simultaneously prevented, attenuated or eliminated.

13. The method of any of claims 1 to 3, wherein the nutritional formula promotes weight loss.

14. The method of any of claims 1 to 3, wherein the nutritional formula promotes fat loss.
